(19) Europäisches Patentamt
European Patent Office
Office européen des brevets

(11) EP 0 761 658 A1

(12) EUROPEAN PATENT APPLICATION

(43) Date of publication:
12.03.1997 Bulletin 1997/11

(21) Application number: 96114020.9

(22) Date of filing: 02.09.1996

(51) Int. Cl.$^6$: C07D 277/46, C07D 263/34, C07D 233/88, A61K 31/17, A61K 31/42, A61K 31/425, A61K 31/40

(84) Designated Contracting States:
AT BE CH DE DK ES FR GB GR IT LI NL PT SE

(30) Priority: 11.09.1995 JP 258107/95
03.03.1996 JP 71206/96

(71) Applicant: NIHON NOHYAKU CO., LTD.
Chuo-ku Tokyo (JP)

(72) Inventors:
- Yamamoto, Kenji
Kawachinagano-shi (JP)
- Kondoh, Kuniaki
Kawachinagano-shi (JP)
- Horiuchi, Kenji
Daito-shi (JP)
- Matsui, Yoshimitsu
Kawachinagano-shi (JP)
- Nagamine, Masashi
Gose-shi (JP)

(74) Representative: Grünecker, Kinkeldey, Stockmair & Schwanhäusser
Anwaltssozietät
Maximilianstrasse 58
80538 München (DE)

# (54) Azole-phenyl urea derivatives as ACAT inhibitors and their production

(57) The present invention relates to azole-phenyl urea derivatives represented by the general formula (I):

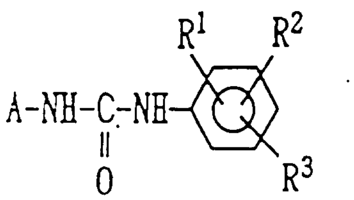

( I )

[wherein each of $R^1$, $R^2$ and $R^3$ is H, halogen, $C_{1-8}$alkyl or the like, and A is a group represented by the formula (i) or (ii):

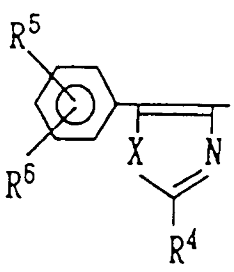

( i )

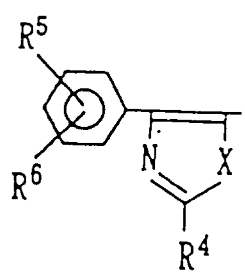

( ii )

(wherein $R^4$, $R^5$, $R^6$ and X are as defined in the specification.)] and a pharmacologically acceptable salt thereof, which have ACAT-inhibitory activity and are useful as a prophylactic and therapeutic agent for hypercholesterolemia, atherosclerosis and various diseases caused by them; a process for producing said derivative; and an ACAT inhibitor contain-

EP 0 761 658 A1

ing said derivative or salt as an active ingredient.

## Description

BACKGROUND OF THE INVENTION

Field of the Invention

The present invention relates to thiazole, oxazole or imidazole derivatives or pharmacologically acceptable salts thereof, which have an excellent inhibitory effect on acyl-CoA:cholesterol O-acyltransferase (ACAT), uses thereof, a process for production thereof, and a method for application thereof.

The azole derivatives represented by the general formula (I) of the present invention and pharmacologically acceptable salts thereof have the effect of reducing serum cholesterol by inhibiting the absorption of cholesterol from intestinal tract and suppress the accumulation of cholesterol esters in the arterial wall. Therefore, they are useful as a prophylactic and therapeutic agent for hypercholesterolemia, atherosclerosis and various diseases caused by them, such as ischemic heart diseases (e.g myocardial infarction) and cerebrovascular diseases (e.g. cerebral infarction and cerebral apoplexy).

Related Art

U.S. Patent 4,113,731 discloses compounds such as N-phenyl-N'-(4-phenyl-2-(p-toluyl)thiazol-5-yl)urea as intermediates for synthesis. Japanese Patent Examined Publication No. 45-6014 discloses compounds such as N-(2,3-diphenylthiazol-4-yl)-N'-phenylurea hydrochloride as anti-inflammatory agents. DE-OS Nos. 2132431 and 2144683 disclose compounds such as N-(4-chlorophenyl)-N'-[4-(3',4'-dimethoxy-6'-methylphenyl)thiazol-2-yl]-N'-methylurea as virucides. Bulletin of the Chemical Society of Japan, 1971, 44(7), 1864-1868 describes compounds such as 2,4-diphenyl-3-methyl-5-phenylcarbamoyliminothiazole as anti-inflammatory agents. Japanese Patent Unexamined Publication No. 48-97868 discloses compounds such as ethyl 4-(4-chlorophenyl)-2-phenylureido-5-thiazoleacetate as antiinflammatory agents. European Journal of Medicinal Chemistry Chimica Therapeutique, 1986, 21(1), 59-64 describes compounds such as N-(4-chlorophenyl)-N'-(4-phenyl-2-thiazolyl)urea as anthelmintics. Journal of Heterocyclic Chemistry, 1988, 25(6), 1821-1824 describes compounds such as N-phenyl-N'-(4-phenyl-2-thiazolyl)urea as anti-inflammatory agents. Journal of Agricultural Food Chemistry, 1989, 37(5), 1438-1441 describes dimethyl 5-(2-thiazolylureido)-1,3-benzenedicarboxylate as an insect growth regulator. Synthesis, 1990, (4), 360-365 describes compounds such as N-[2-(4-methylphenyl)-4-trifluoromethylthiazol-5-yl]-N'-phenylurea as anti-inflammatory agents. Indian Journal of Heterocyclic Chemistry, 1994, 4(1), 59-62 describes compounds such as N-(3,5-dimethoxyphenyl)-N'-(4-phenylthiazol-2-yl)urea as anti-inflammatory agents. Journal of Medicinal Chemistry, 1971, 14(11), 1075-1077 describes compounds such as N-(4-chlorophenyl)-N'-(4,5-diphenyl-2-oxazolyl)urea as anti-inflammatory agents. Japanese Patent Unexamined Publication No. 60-1173 and Chemical & Pharmaceutical Bulletin, 1984, 32(3), 1032-1039 describe compounds such as N-phenyl-N'-(5-phenyl-2-oxazolyl)urea as aldose reductase inhibitors. Journal of Medicinal Chemistry, 1969, 12(5), 1010-1015 describes compounds such as N-(2-imidazolyl)-N'-phenylurea as immunosuppressants. Japanese Patent Unexamined Publication No. 2-28264 discloses imidazole-urea derivatives as thermal transfer inks. Japanese Patent Unexamined Publication Nos. 1-295257 and 3-219242 disclose imidazoleurea derivatives as photographic materials. However, these references do not describe the above compounds as having ACAT-inhibitory effect or serum-cholesterol-lowering activity, at all. Further, U.S. Patent 5,162,360 discloses thiazole derivatives having ACAT-inhibitory effect and European Patent No. 477778 discloses imidazole derivatives having ACAT-inhibitory effect. However, the effect of all the thiazole and imidazole derivatives are not sufficient.

SUMMARY OF THE INVENTION

The present invention provides novel thiazole, oxazole or imidazole derivatives or pharmacologically acceptable salts thereof, ACAT inhibitors containing the derivative or the salt as an active ingredient, a process for production of the derivatives, and a method for application of the derivatives.

DETAILED DESCRIPTION OF THE INVENTION

The present inventors found that a novel compound not known in any literature, an azole derivative represented by the general formula (I):

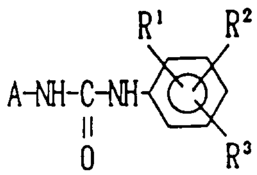

(I)

[wherein $R^1$, $R^2$ and $R^3$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different, and A is a group represented by the formula (i):

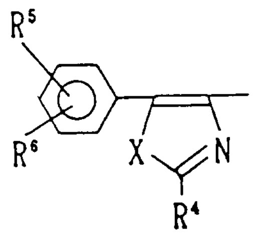

(i)

(wherein $R^4$ is a $C_{1-8}$alkyl group, a $C_{3-8}$cycloalkyl group, a mono-$C_{1-8}$alkylamino group, a di-$C_{1-8}$alkylamino group whose $C_{1-8}$alkyl groups may be the same or different, an aliphatic cyclic amino group which may be substituted by one or more $C_{1-8}$alkyl groups, an unsubstituted phenyl group, or a substituted phenyl group having one or two substituents which may be the same or different and are selected from the group consisting of halogen atoms and $C_{1-8}$alkyl groups; $R^5$ and $R^6$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different; and X is -O-, -S- or -N($R^7$)-(wherein $R^7$ is a hydrogen atom; a $C_{1-8}$alkyl group; an unsubstituted phenyl-$C_{1-8}$alkyl group; a substituted phenyl-$C_{1-8}$alkyl group having one or more substituents which may be the same or different and are selected from the group consisting of halogen atoms, $C_{1-8}$alkyl groups and $C_{1-8}$alkoxy groups; a (2-trimethylsilyl)ethoxymethyl group; or a substituted $C_{1-8}$alkyl group having one or more $C_{1-8}$alkoxy groups as the substituent(s))) or a group represented by the formula (ii):

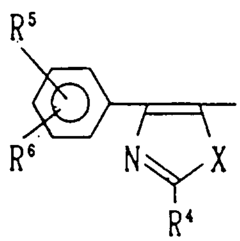

(ii)

(wherein $R^4$, $R^5$, $R^6$ and X are as defined above), provided that when A is a group represented by the formula (ii), there is excluded the case wherein X is -S-, each of $R^1$, $R^2$, $R^3$, $R^5$ and $R^6$ is a hydrogen atom, and $R^4$ is a $C_{1-8}$alkyl group, an unsubstituted phenyl group or a substituted phenyl group having one or two $C_{1-8}$alkyl groups as the substituent(s)] or a pharmacologically acceptable salt thereof has ACAT-inhibitory activity much higher than that of well-known azole derivatives and is useful as a serum cholesterol lowering agent or an agent for curing arteriosclerosis, whereby the present invention has been accomplished.

In the general formula (I) in the present invention, the halogen atoms include chlorine atom, fluorine atom, iodine atom, bromine atom, etc. The term "$C_{1-8}$alkyl group" means a linear or branched alkyl group having 1 to 8 carbon atoms, such as methyl, ethyl, n-propyl, isopropyl, n-butyl, isobutyl, sec-butyl, tert-butyl, n-pentyl, isopentyl, neopentyl, 1-methylbutyl, 2-methylbutyl, 1,2-dimethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1,1-dimethylpentyl, 1,2-dimethylpentyl, 1,3-dimethylpentyl, 1,4-dimethylpentyl, 2,2-dimethylpentyl, 2,3-dimethylpentyl, 2,4-dimethylpentyl, 3,3-dimethylpentyl, 3,4-dimethylpentyl, 1-ethylpentyl, 2-ethylpentyl, 3-ethylpentyl, 1,1,2-trimethylbutyl, 1,1,3-trimethylbutyl, 1,2,2-trimethylbutyl, 2,2,3-trimethylbutyl, 1-ethyl-1-methylbutyl, 1-ethyl-2-methylbutyl, 1-ethyl-3-methylbutyl, 1-propylbutyl, 1-isopropylbutyl, n-octyl, 6-methylheptyl or the like.

The term "$C_{1-8}$alkoxy group" means a linear or branched alkoxy group having 1 to 8 carbon atoms, such as methoxy, ethoxy, n-propoxy, isopropoxy, n-butoxy, isobutoxy, sec-butoxy, tert-butoxy, n-pentyloxy, isopentyloxy, neopentyloxy, n-hexyloxy, n-heptyloxy, n-octyloxy or the like. The term "mono-$C_{1-8}$alkylamino group" means a group such as methylamino, ethylamino, n-propylamino, isopropylamino, n-butylamino, isobutylamino, sec-butylamino, tert-butylamino, n-pentylamino, isopentylamino, neopentylamino, n-hexylamino, n-heptylamino, n-octylamino or the like. The term "di-$C_{1-8}$alkylamino group" means a group such as dimethylamino, diethylamino, di-n-propylamino, diisopropylamino, methylethylamino, di-n-butylamino, diisobutylamino, di-sec-butylamino, di-tert-butylamino, methyl-n-propylamino, di-n-pentylamino, methyl-n-butylamino, methyl-n-pentylamino, di-n-hexylamino, methyl-n-hexylamino, di-n-heptylamino, methyl-n-heptylamino, di-n-octylamino, methyl-n-octylamino, ethyl-n-propylamino, ethyl-n-butylamino or the like.

The term "halo-$C_{1-8}$alkyl group" means a group such as chloromethyl, fluoromethyl, bromomethyl, iodomethyl, dichloromethyl, difluoromethyl, trichloromethyl, trifluoromethyl, 1,1,1-trifluoroethyl or the like. The term "$C_{1-8}$alkylthio group" means a group such as methylthio, ethylthio, n-propylthio, isopropylthio, n-butylthio, isobutylthio, sec-butylthio, tert-butylthio, n-pentylthio, isopentylthio, neopentylthio, n-hexylthio, n-heptylthio, n-octylthio or the like. The term "$C_{3-8}$cycloalkyl group" means a group such as cyclopropyl, cyclobutyl, cyclopentyl, cyclohexyl, cycloheptyl, cyclooctyl or the like. The term "unsubstituted aliphatic cyclic amino group or substituted aliphatic cyclic amino group having one or more $C_{1-8}$alkyl groups as the substituent(s)" means a group such as pyrrolidino, piperidino, morpholino, 2-methylmorpholino, 2,6-dimethylmorpholino, thiomorpholino, piperazino, N-methylpiperazino or the like. The term "phenyl-$C_{1-8}$alkyl group" means a group such as benzyl, phenethyl or the like.

Preferable substituents are as follows. When X is -O- or -S-, each of $R^1$, $R^2$ and $R^3$ is a hydrogen atom, a methyl group, an ethyl group, a n-propyl group, an isopropyl group, a n-butyl group, a sec-butyl group, an isobutyl group or a tert-butyl group, $R^4$ is a methyl group, an ethyl group, a n-propyl group, an isopropyl group, a n-butyl group, a sec-butyl group, an isobutyl group, a tert-butyl group or a phenyl group, each of $R^5$ and $R^6$ is a hydrogen atom, a chlorine atom, a methyl group, an ethyl group, a n-propyl group, an isopropyl group, a n-butyl group, a sec-butyl group, an isobutyl group, a tert-butyl group, a methylthio group, an ethylthio group, a n-propylthio group, an isopropylthio group, a n-butylthio group, a sec-butylthio group, an isobutylthio group or a tert-butylthio group.

When X is -N($R^7$), each of $R^1$, $R^2$ and $R^3$ is a hydrogen atom, a methyl group, an ethyl group, a n-propyl group, an isopropyl group, a n-butyl group, a sec-butyl group, an isobutyl group or a tert-butyl group, $R^4$ is a phenyl group, each of $R^5$ and $R^6$ is a hydrogen atom, a chlorine atom, a methyl group, an ethyl group, a n-propyl group, an isopropyl group, a n-butyl group, a sec-butyl group, an isobutyl group, a tert-butyl group, a methylthio group, an ethylthio group, a n-propylthio group, an isopropylthio group, a n-butylthio group, a sec-butylthio group, an isobutylthio group or a tert-butylthio group, and $R^7$ is a benzyl group.

Typical examples of the salt of the azole derivative represented by the general formula (I) of the present invention are inorganic acid salts such as hydrochloride, nitrate, sulfate, hydrobromate, hydroiodate, perchlorate, phosphate, etc.; sulfonates such as methanesulfonate, benzenesulfonate, p-toluenesulfonate, etc.; organic acid salts such as fumarate, succinate, citrate, acetate, tartrate, oxalate, maleate, etc.; and amino acid salts such as glutamate, aspartate, etc. Preferable examples of the salt are hydrochloride and p-toluenesulfonate.

The azole derivative of the general formula (I) of the present invention can be produced by the following process A or process B.

Process A

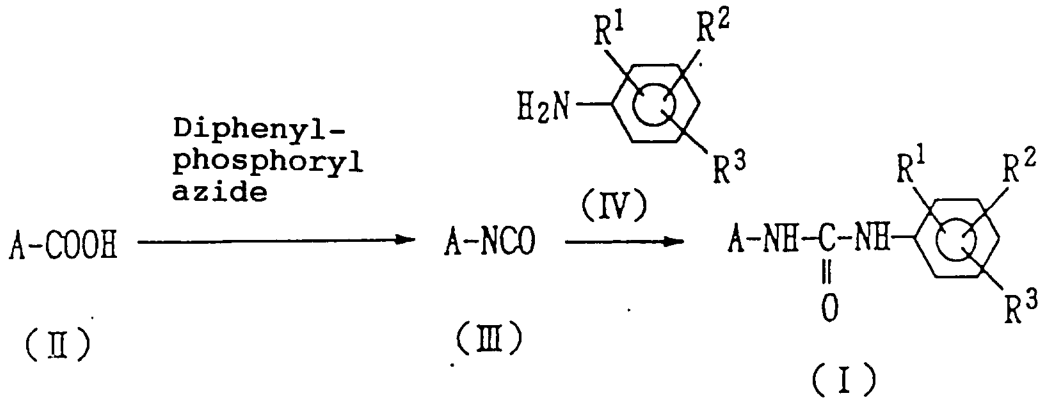


wherein A, $R^1$, $R^2$ and $R^3$ are as defined above.

In detail, the azole derivative of the general formula (I) can be produced by reacting a compound of the general formula (II) with diphenylphosphoryl azide in the presence of an organic amine such as triethylamine in an inert solvent such as benzene, toluene, xylene, fluorobenzene or dioxane in a temperature range of room temperature to about 150°C to obtain an isocyanate of the general formula (III), and then reacting the isocyanate with a compound of the general formula (IV) in a temperature range of room temperature to about 150°C after or without isolating the isocyanate.

Since the reactions are equimolar reactions, it is sufficient that the reactants for carrying out each reaction are used in equimolar amounts, though either of them may be used in excess.

Process B

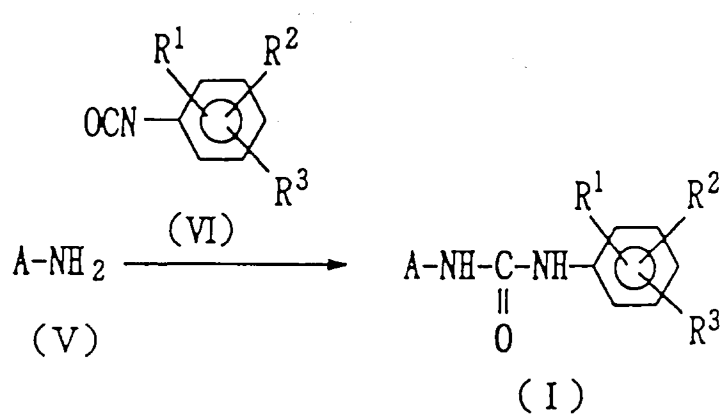

wherein A, $R^1$, $R^2$ and $R^3$ are as defined above.

In detail, the azole derivative of the general formula (I) can be produced by reacting a compound of the general formula (V) with a compound of the general formula (VI) in a solvent such as benzene, toluene, xylene, fluorobenzene, dioxane, tetrahydrofuran or ethyl acetate in a temperature range of room temperature to about 150°C.

Since the reaction is an equimolar reaction, it is sufficient that the reactants for carrying out the reaction are used in equimolar amounts, though either of them may be used in excess.

The compound of the general formula (II) used in process A can be produced by any of the following processes C to G.

Process C (in the case where X = S)

A compound of the general formula (IIa) or (IIb) can be produced from a compound of the general formula (VII) or (VIII), respectively, by the process described in Liebigs Annalen der Chemie, p. 250 (1889) or a process based thereon.

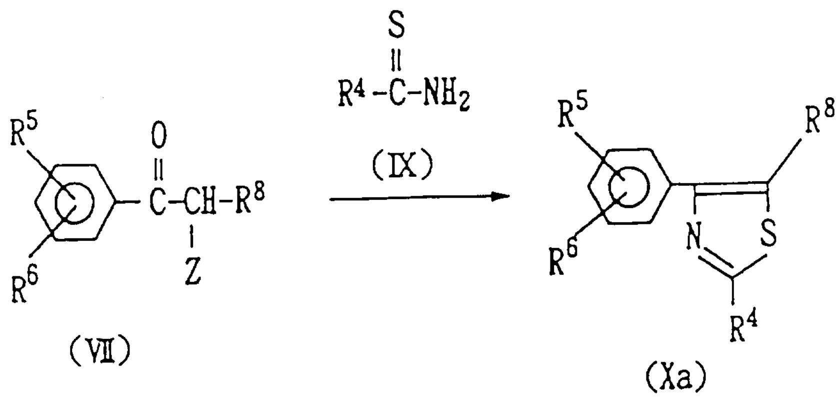

(Xa)

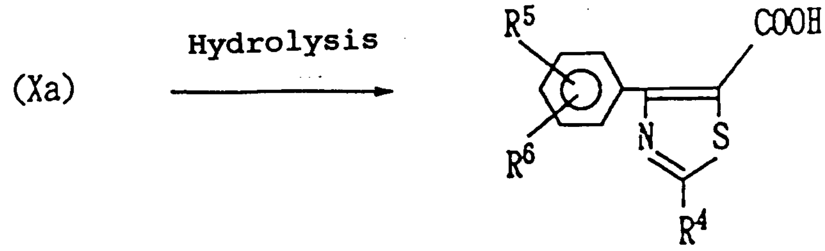


(IIa)

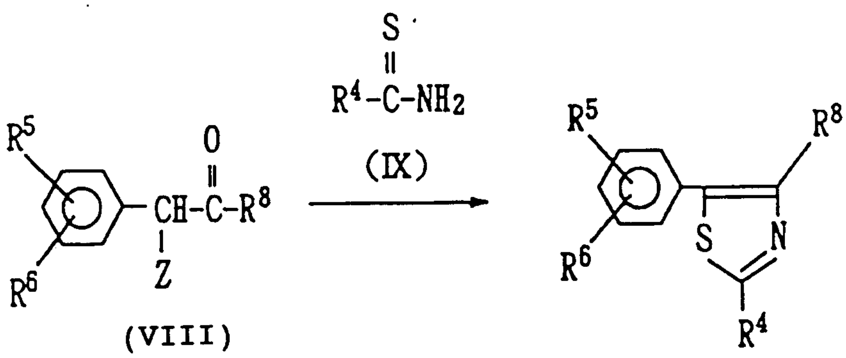

(IX)

(VIII)

(Xb)

(Xb)

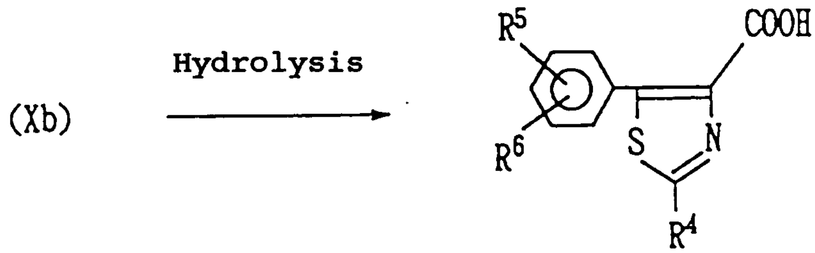


(IIb)

wherein $R^4$, $R^5$ and $R^6$ are as defined above, $R^8$ is $COOR^9$ (wherein $R^9$ is a $C_{1-4}$ alkyl group) or a nitrile group, and Z is a halogen atom.

In detail, a compound of the general formula (VII) or (VIII) is reacted with a thioamide or thiourea (IX), respectively, to be converted into a thiazole (Xa) or (Xb), respectively. Then, the thiazole (Xa) or (Xb) is hydrolyzed, whereby a carboxylic acid of the general formula (IIa) or (IIb), respectively, can be produced.

The conversion of the compound of the general formula (VII) or (VIII) into the compound of the general formula (Xa) or (Xb), respectively, can be carried out by reacting the compound of the general formula (VII) or (VIII) with the compound of the general formula (IX) in an ordinary solvent (e.g. methanol, ethanol, isopropanol or dioxane) in a temperature range of room temperature to about 100°C.

The conversion of the compound of the general formula (Xa) or (Xb) into the compound of the general formula (IIa) or (IIb), respectively, can be carried out by using either an aqueous solution of an alkali metal hydroxide (e.g. sodium hydroxide or potassium hydroxide) or a mineral acid (e.g. hydrochloric acid, sulfuric acid or hydrobromic acid). As a solvent for this reaction, methanol, ethanol, isopropanol, dioxane, etc. are suitable. The reaction may be carried out without any solvent. The reaction temperature ranges preferably from room temperature to about 120°C.

Process D (in the case where A = a group of the formula (ii), and X = S)

The compound of the general formula (IIa) can be produced from a compound of the general formula (XI) by the process described in Zeitschrift fuer Chemie, Vol. 14, p. 470 (1974) or a process based thereon.

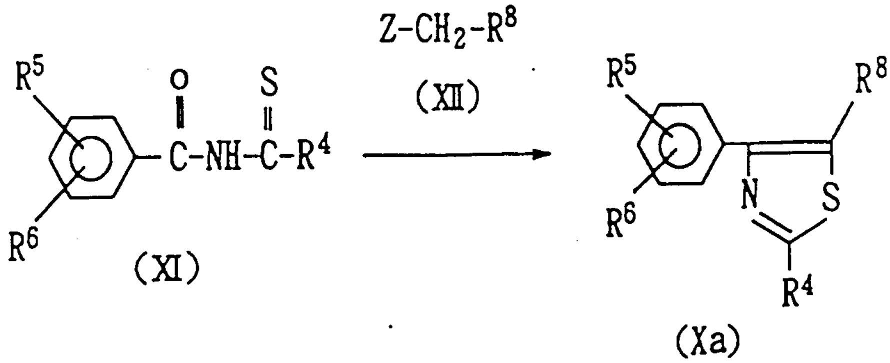


(XI) (Xa)

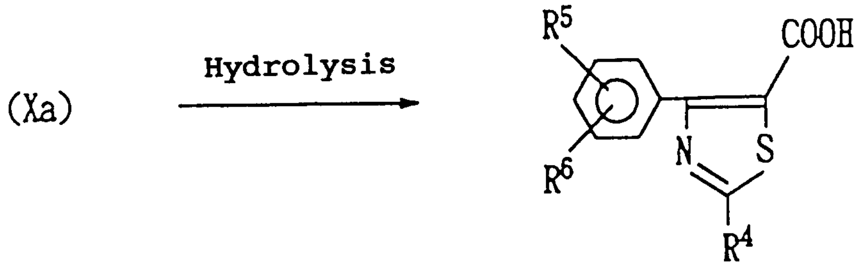


(IIa)

wherein $R^4$, $R^5$, $R^6$, $R^8$ and Z are as defined above.

In detail, a compound of the general formula (XI) is reacted with a compound of the general formula (XII) to be converted into a thiazole of the general formula (Xa), after which the thiazole of the general formula (Xa) is hydrolyzed, whereby a carboxylic acid of the general formula (IIa) can be produced.

The conversion of the compound of the general formula (XI) into the compound of the general formula (Xa) can be carried out by reacting the compound of the general formula (XI) with the compound of the general formula (XII) in the presence of an organic base such as triethylamine, pyridine, piperazine or piperidine in an ordinary solvent (e.g. methanol, ethanol, iso-propanol, n-butanol or dioxane) in a temperature range of room temperature to about 100°C.

The conversion of the compound of the general formula (Xa) into the compound of the general formula (IIa) can be carried out by using either an aqueous solution of an alkali metal hydroxide (e.g. sodium hydroxide or potassium hydroxide) or a mineral acid (e.g. hydrochloric acid, sulfuric acid or hydrobromic acid).

As a solvent for this reaction, methanol, ethanol, isopropanol, dioxane, etc. are suitable. The reaction may be carried out without any solvent. The reaction temperature ranges preferably from room temperature to about 120°C.

Process E (in the case where A = a group of the formula (i), and X = O)

A compound of the general formula (IIc) can be produced from a compound of the general formula (XIII) by the process described in Yakugaku Zasshi, Vol. 82, p. 136 (1962) or a process based thereon.

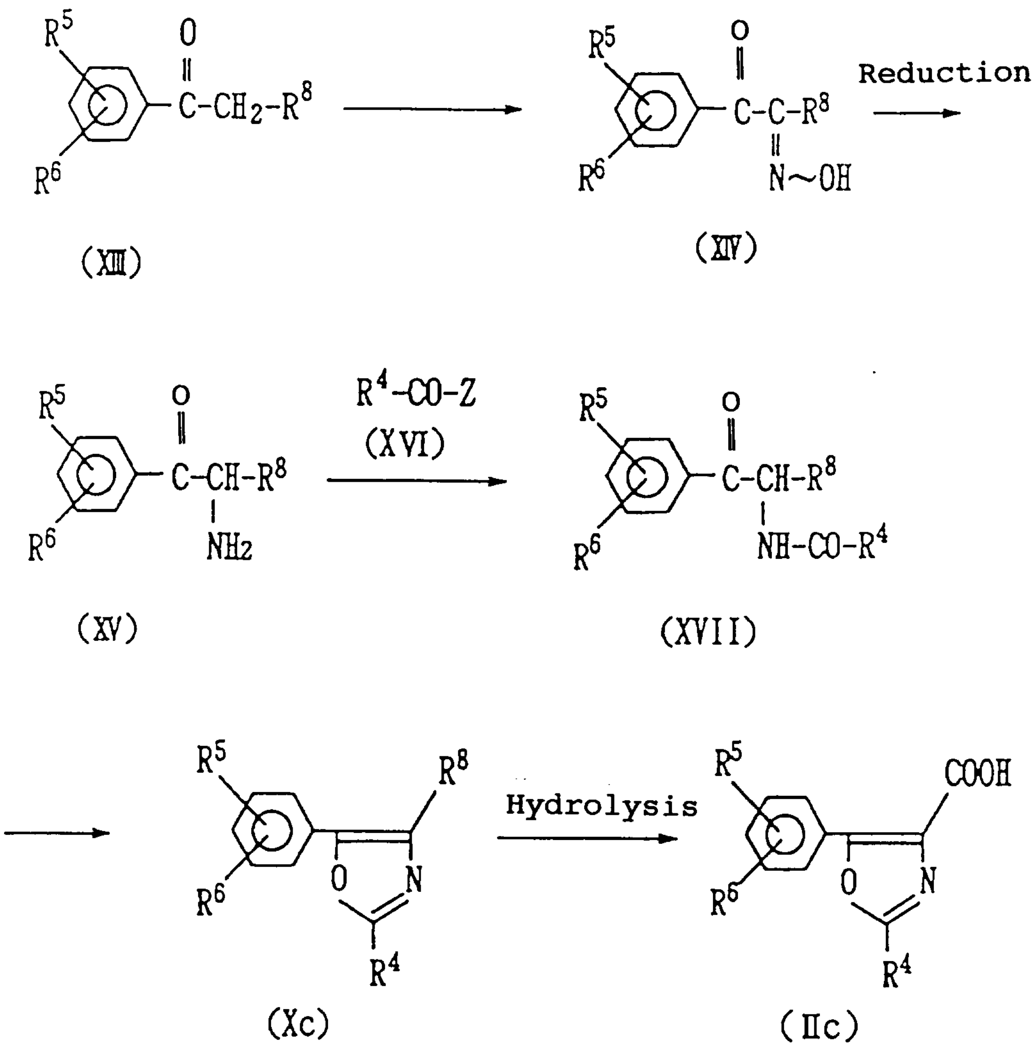


(XIII) (XIV) (XV) (XVII) (Xc) (IIc)

wherein $R^4$, $R^5$, $R^6$, $R^8$ and Z are as defined above.

In detail, a compound of the general formula (XIII) is converted into an oxime, i.e., a compound of the general formula (XIV), which is reduced into an amine of the general formula (XV). Then, the amine is reacted with an acid halide of the general formula (XVI) to be converted into an amide of the general formula (XVII). The compound of the general formula (XVII) is cyclized into an oxazole of the general formula (Xc), which is hydrolyzed, whereby a compound of the general formula (IIc) can be produced.

The conversion of the compound of the general formula (XIII) into the compound of the general formula (XIV) can be carried out by the use of a nitrite (e.g. sodium nitrite) in a solvent such as an organic acid (e.g. acetic acid) or a mineral acid (e.g. hydrochloric acid or sulfuric acid) in a temperature range of room temperature to about 120°C.

The conversion of the compound of the general formula (XIV) into the amine of the general formula (XV) can be carried out under acidic conditions by the use of zinc, iron, tin, tin chloride or the like in a solvent (e.g. acetic acid, ethanol, methanol or isopropanol) in a temperature range of room temperature to about 120°C.

The conversion of the amine of the general formula (XV) into the compound of the general formula (XVII) can be carried out in the presence of an acid halide of the general formula (XVI) and a base (e.g. potassium carbonate or sodium acetate) in a solvent (e.g. water or dioxane) in a temperature range of room temperature to about 100°C.

The conversion of the amide of the general formula (XVII) into the oxazole of the general formula (Xc) can be carried out in the presence of phosphoryl chloride, thionyl chloride or the like in an inert solvent (e.g. benzene, toluene or xylene) in a temperature range of room temperature to about 120°C.

The conversion of the oxazole of the general formula (Xc) into the compound of the general formula (IIc) can be carried out by using either an aqueous solution of an alkali metal hydroxide (e.g. sodium hydroxide or potassium hydroxide) or a mineral acid (e.g. hydrochloric acid, sulfuric acid or hydrobromic acid).

As a solvent for this reaction, methanol, ethanol, isopropanol, dioxane, etc. are suitable. The reaction may be carried out without any solvent. The reaction temperature ranges preferably from room temperature to about 120°C.

Process F (in the case where A = a group of the formula (ii), and X = O)

A compound of the general formula (IId) can be produced from a compound of the general formula (VII) by the process described in Yakugaku Zasshi, Vol. 82, p. 140 (1962) or a process based thereon.

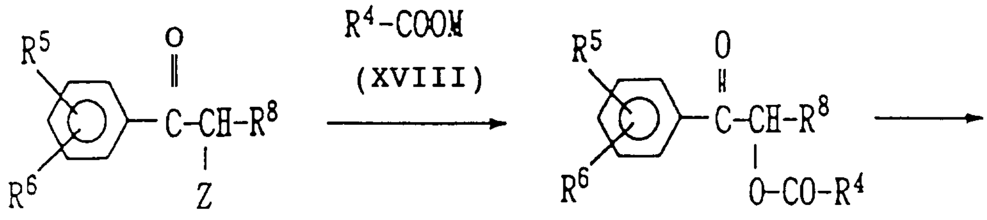


(VII) (XIX)

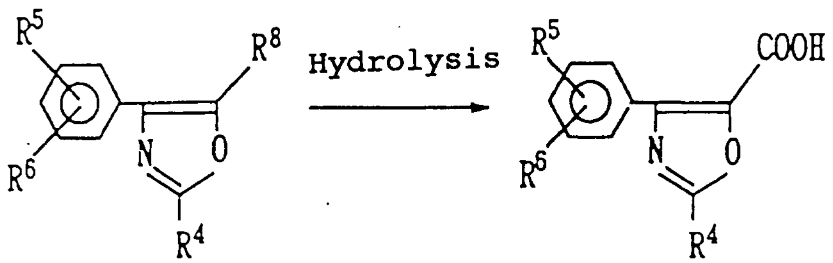


(Xd) (IId)

wherein $R^4$, $R^5$, $R^6$, $R^8$ and Z are as defined above, and M is a sodium or potassium atom.

In detail, a compound of the general formula (VII) is reacted with a carboxylate of the general formula (XVIII) to be converted into an ester of the general formula (XIX), which is cyclized into an oxazole of the general formula (Xd), followed by hydrolysis, whereby a compound of the general formula (IId) can be produced.

The conversion of the compound of the general formula (VII) into the ester of the general formula (XIX) can be carried out by reacting the compound of the general formula (VII) with the carboxylate of the general formula (XVIII) in a solvent (e.g. ethanol or methanol) in a temperature range of room temperature to about 100°C. The conversion of the ester of the general formula (XIX) into the oxazole of the general formula (Xd) can be carried out in the presence of an ammonium salt (e.g. ammonium acetate or ammonium formate) in a solvent (e.g. acetic acid or formic acid) in a temperature range of room temperature to about 120°C.

The conversion of the oxazole of the general formula (Xd) into the compound of the general formula (IId) can be carried out by using either an aqueous solution of an alkali metal hydroxide (e.g. sodium hydroxide or potassium hydroxide) or a mineral acid (e.g. hydrochloric acid, sulfuric acid or hydrobromic acid). As a solvent for this reaction, methanol, ethanol, isopropanol, dioxane, etc. are suitable. The reaction may be carried out without any solvent. The reaction temperature ranges preferably from room temperature to about 120°C.

Process G (in the case where X = $-N(R^7)-$)

A compound of the general formula (IIf) or (IIg) can be produced from a compound of the general formula (XX) by the process described in Tetrahedron Letters, Vol. 35, No. 11, pp. 1635-1638 (1994) or a process based thereon.

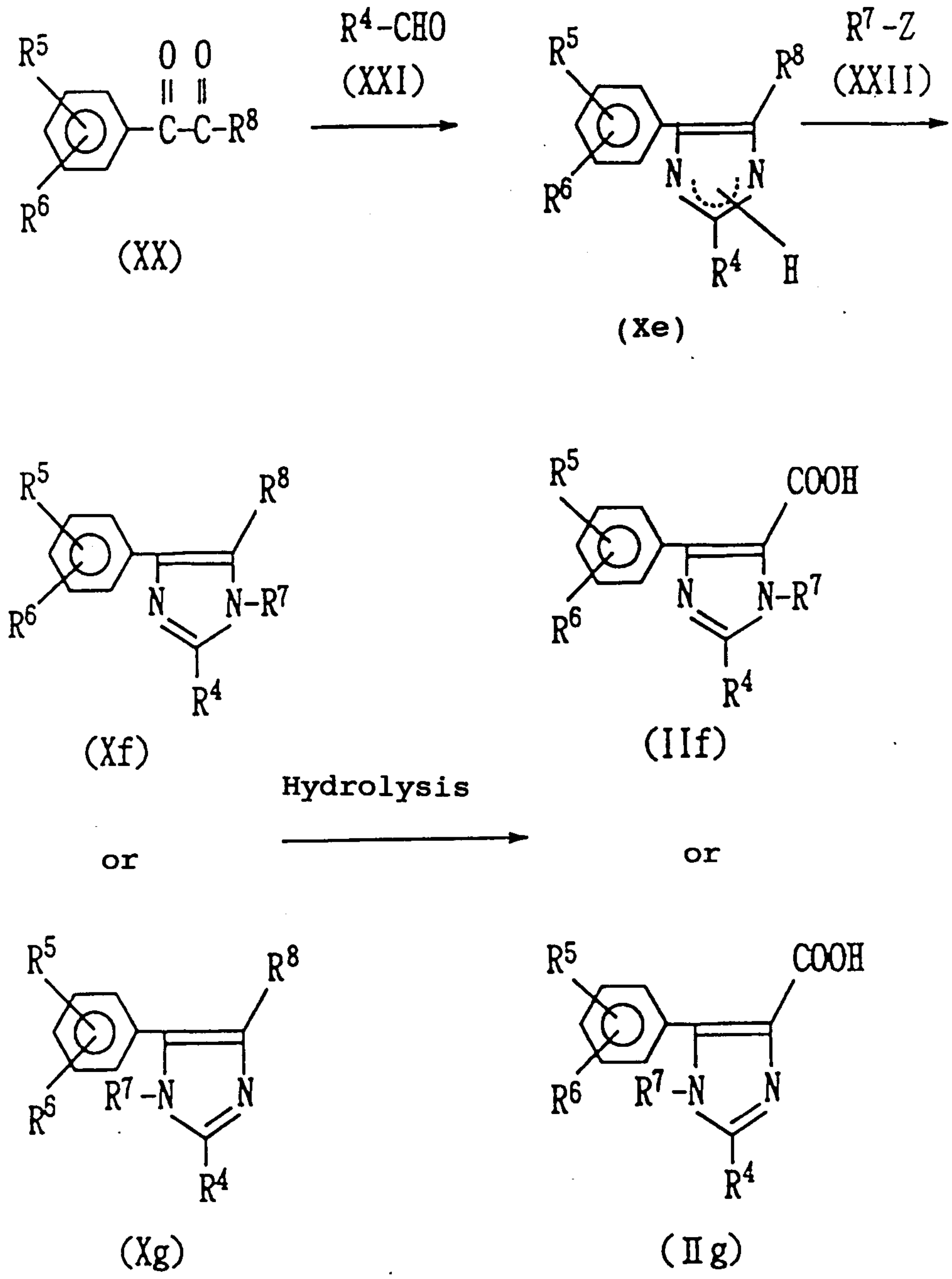


(XX) (Xe)

(Xf) (IIf)

or or

(Xg) (IIg)

wherein $R^4$, $R^5$, $R^6$, $R^7$, $R^8$ and Z are as defined above.

In detail, a compound of the general formula (XX) is reacted with an aldehyde of the general formula (XXI) to be converted into an imidazole of the general formula (Xe), which is alkylated into a compound of the general formula (Xf) or (Xg) with an alkyl halide of the general formula (XXII). Then, the compound of the general formula (Xf) or (Xg) is hydrolyzed, whereby a compound of the general formula (IIf) or (IIg), respectively, can be produced.

The conversion of the compound of the general formula (XX) into the imidazole of the general formula (Xe) can be carried out by reacting the compound of the general formula (XX) with the aldehyde of the general formula (XXI) in the presence of an ammonium salt (e.g. ammonium acetate or ammonium formate) in a solvent (e.g. acetic acid or formic acid) in a temperature range of room temperature to about 120°C.

The conversion of the imidazole of the general formula (Xe) into the compound of the general formula (Xf) or (Xg) can be carried out by reacting the imidazole of the general formula (Xe) with the alkyl halide of the general formula (XXII) in the presence of a base (e.g. potassium carbonate and sodium hydride) in a solvent (e.g. dimethylformamide, tetrahydrofuran or acetone) in a temperature range of room temperature to about 120°C.

The conversion of the compound of the general formula (Xf) or (Xg) into the compound of the general formula (IIf)

or (IIg), respectively, can be carried out by using either an aqueous solution of an alkali metal hydroxide (e.g. sodium hydroxide or potassium hydroxide) or a mineral acid (e.g. hydrochloric acid, sulfuric acid or hydrobromic acid). As a solvent for this reaction, methanol, ethanol, isopropanol, dioxane, etc. are suitable. The reaction may be carried out without any solvent. The reaction temperature ranges preferably from room temperature to about 120°C.

The compound of the general formula (V) used in process B can be produced by any of the following processes H to L.

Process H (in the case where A = a group of the formula (i), and X = S)

A compound of the general formula (Va) can be produced from a compound of the general formula (XXIII) by the process described in Journal of American Chemical Society, Vol. 77, pp. 5444-5445 (1955) or a process base thereon.

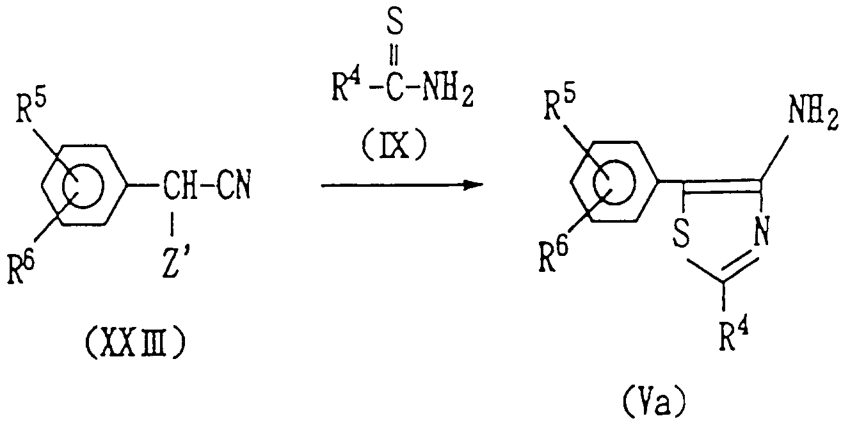
(XXIII)
(IX)
(Va)

wherein $R^4$, $R^5$ and $R^6$ are as defined above, and Z' is a halogen atom or $-OSO_2R^{10}$ (wherein $R^{10}$ is a $C_{1-4}$ alkyl group, a phenyl group or a p-toluyl group).

In detail, a thiazole of the general formula (Va) can be produced by reacting a compound of the general formula (XXIII) with a thioamide or thiourea of the general formula (IX) in an ordinary solvent (e.g. methanol, ethanol, isopropanol or dioxane) in a temperature range of room temperature to about 100°C.

Process I (in the case where A = a group of the formula (ii), and X = S)

A compound of the general formula (Vb) can be produced from a compound of the general formula (XXIV) by the process described in Journal fuer Praktische Chemie, Vol. 316, pp. 299-303 (1974) or a process based thereon.

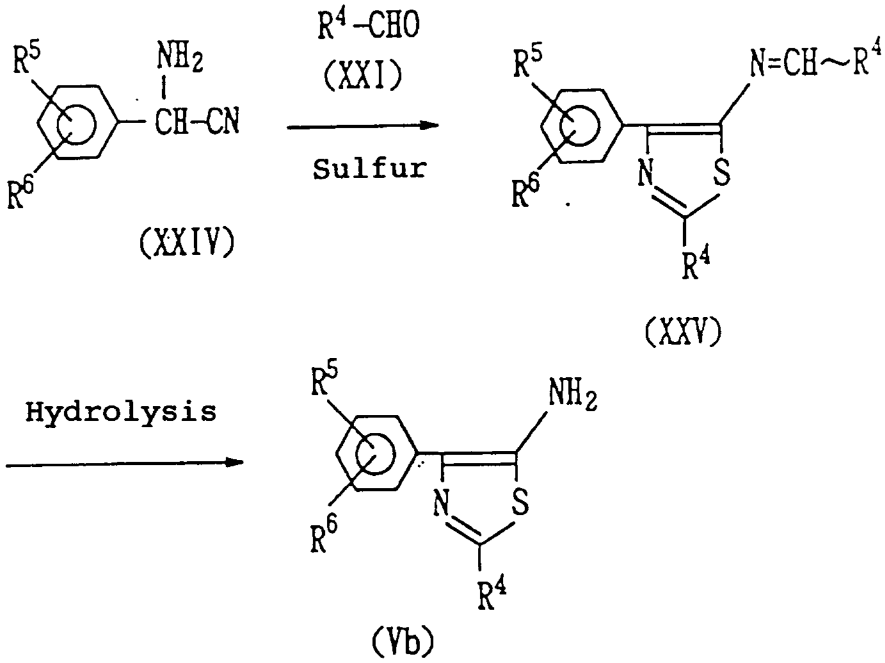


(XXIV)

(XXV)

(Vb)

wherein $R^4$, $R^5$ and $R^6$ are as defined above.

In detail, a compound of the general formula (XXIV) is reacted with an aldehyde of the general formula (XXI) and sulfur to be converted into a thiazole of the general formula (XXV), which is hydrolyzed, whereby a compound of the general formula (Vb) can be produced.

The conversion of the compound of the general formula (XXIV) into the thiazole of the general formula (XXV) can be carried out by reacting the compound of the general formula (XXIV) with the aldehyde of the general formula (XXI) and sulfur in the presence of an organic amine (e.g. triethylamine) in a solvent (e.g. benzene or toluene) in a temperature range of room temperature to about 100°C.

The conversion of the thiazole of the general formula (XXV) into the compound of the general formula (Vb) can be carried out by using a mineral acid (e.g. hydrochloric acid, sulfuric acid or hydrobromic acid). As a solvent for this reaction, methanol, ethanol, isopropanol, dioxane, etc. are suitable. The reaction may be carried out without any solvent. The reaction temperature ranges preferably from room temperature to about 120°C.

Process J (in the case where A = a group of the formula (ii), and X = O)

A compound of the general formula (Vc) can be produced from a compound of the general formula (XXIV) by the process described in Bulletin de la Societe Chemique de france, Vol. 12, p. 4619 (1967) or a process based thereon.

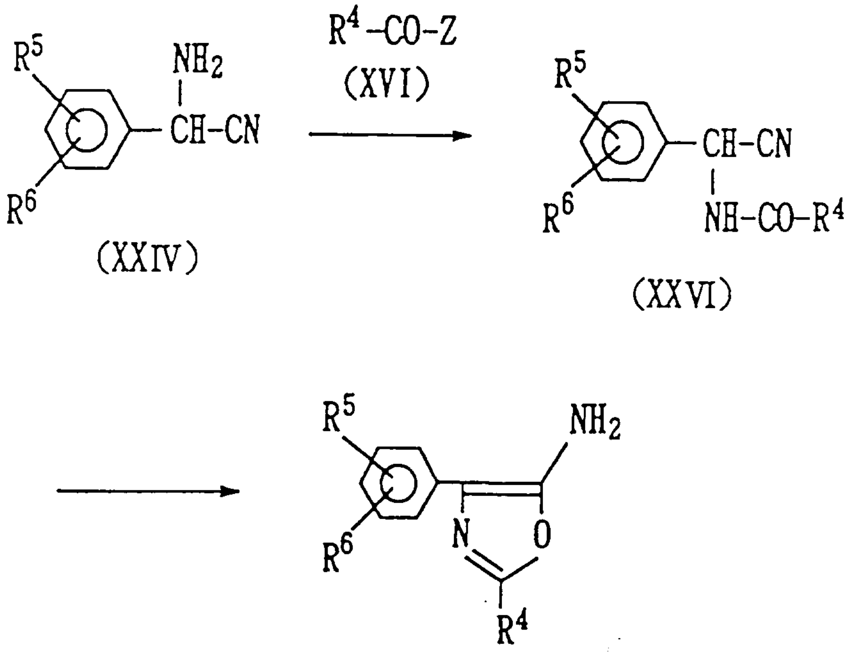


(XXIV)

(XXVI)

(Vc)

wherein $R^4$, $R^5$, $R^6$ and Z are as defined above.

In detail, a compound of the general formula (XXIV) is reacted with an acid halide of the general formula (XVI) to be converted into an amide of the general formula (XXVI), which is cyclized, whereby an oxazole of the general formula (Vc) can be produced.

The conversion of the compound of the general formula (XXIV) into the amide of the general formula (XXVI) can be carried out by reacting the compound of the general formula (XXIV) with the acid halide of the general formula (XVI) in the presence of an organic amine such as triethylamine in a solvent (e.g. benzene, toluene, dichloromethane or tetrahydrofuran) in a temperature range of room temperature to about 100°C.

The conversion of the amide of the general formula (XXVI) into the oxazole of the general formula (Vc) can be carried out by reacting the amide of the general formula (XXVI) by the use of a mineral acid (e.g. hydrochloric acid or sulfuric acid) in a solvent (e.g. benzene, toluene, dichloromethane or chloroform) in a temperature range of room temperature to about 100°C.

Process K (in the case where A = a group of the formula (i), and X = O)

A compound of the general formula (Vd) can be produced from a compound of the general formula (XXVII) by the process described in Journal of Heterocyclic Chemistry, Vol. 25, p. 1413 (1988) or a process based thereon.

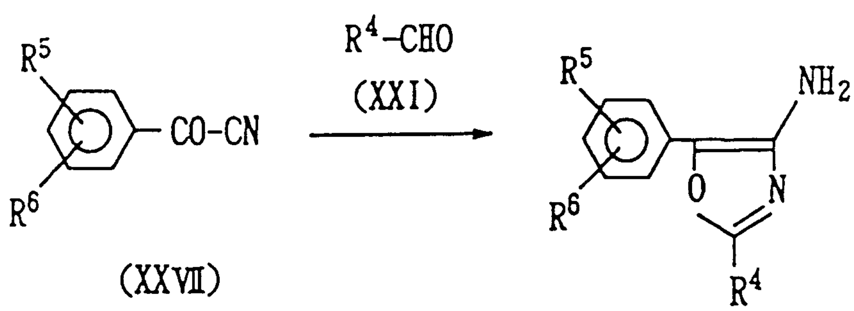


(XXVII)

(Vd)

wherein $R^4$, $R^5$ and $R^6$ are as defined above.

In detail, an oxazole of the general formula (Vd) can be produced by reacting a compound of the general formula (XXVII) with an aldehyde of the general formula (XXI) in the presence of an ammonium salt (e.g. ammonium acetate or ammonium formate) in a solvent (e.g. acetic acid or formic acid) in a temperature range of room temperature to about 100°C.

Process L (in the case where X = -N($R^7$)-)

A compound of the general formula (Ve) or (Vf) can be produced from a compound of the general formula (XXVIII) by the process described in Gazzetta Chimica Italiana, Vol. 85, No. 208, p. 1329 (1955) or a process based thereon.

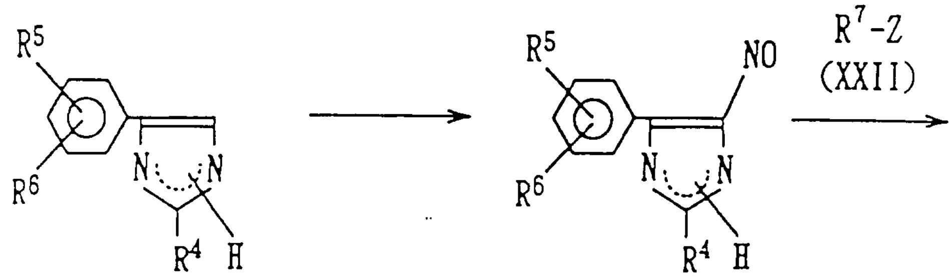


(XXVIII) (XXIX)

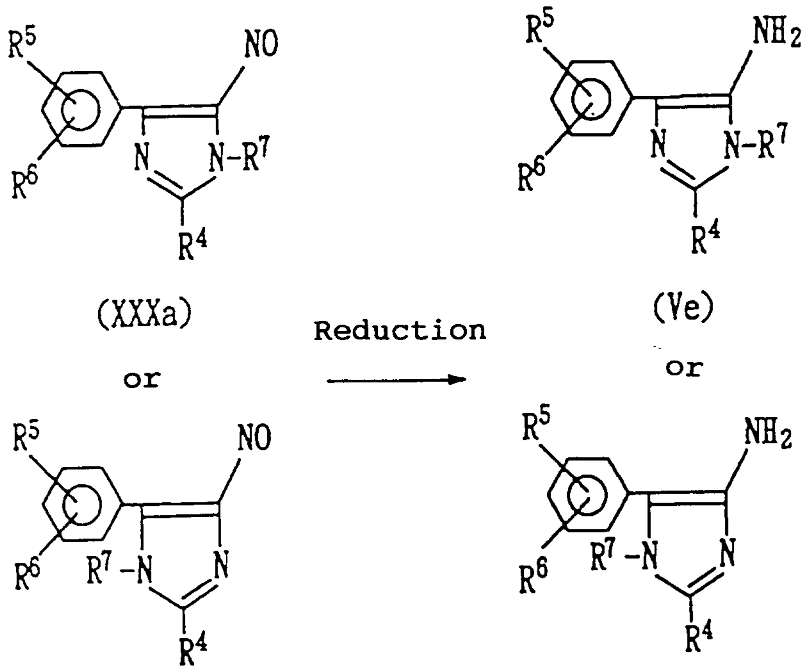


(XXXa) or (XXXb)

(Ve) or (Vf)

wherein $R^4$, $R^5$, $R^6$, $R^7$ and Z are as defined above.

In detail, an imidazole of the general formula (XXVIII) is subjected to nitrosation into a nitroso compound of the general formula (XXIX), which is alkylated into a compound of the general formula (XXXa) or (XXXb) with an alkyl halide of the general formula (XXII). Then, the compound of the general formula (XXXa) or (XXXb) is reduced, whereby a compound of the general formula (Ve) or (Vf), respectively, can be produced.

The conversion of the imidazole of the general formula (XXVIII) into the nitroso compound of the general formula (XXIX) can be carried out by reacting the imidazole of the general formula (XXVIII) with a nitrite such as sodium nitrite in a solvent such as acetic acid in a temperature range of room temperature to about 120°C.

The conversion of the nitroso compound of the general formula (XXIX) into the compound of the general formula (XXXa) or (XXXb) can be carried out by reacting the nitroso compound of the general formula (XXIX) with the alkyl halide of the general formula (XXII) in the presence of a base (e.g. potassium carbonate or sodium hydride) in a solvent (e.g. dimethylformamide, tetrahydrofuran or acetone) in a temperature range of room temperature to about 120°C.

The conversion of the compound of the general formula (XXXa) or (XXXb) into the compound of the general formula (Ve) or (Vf), respectively, can be carried out by using any of various reducing agents. Preferable examples of method for this conversion are a method of reducing the compound of the general formula (XXXa) or (XXXb) with zinc, iron, tin, tin chloride or the like under acidic conditions in a solvent (e.g. acetic acid, ethanol, methanol or isopropanol) in a temperature range of room temperature to about 120°C, and a method of hydrogenating the compound of the general formula (XXXa) or (XXXb) in the presence of a metal catalyst (e.g. platinum oxide, nickel or palladium) in a solvent (e.g. ethanol, acetic acid or ethyl acetate) in a temperature range of room temperature to about 120°C in a pressure range of atmospheric pressure to about 5 atmospheres.

Specific examples of azole derivatives of the general formula (I) obtained by the above production processes are given in Table 1 but they are not intended in any way to limit the scope of the present invention.

The abbreviations in Table 1 stand for the following substituents:

Ph: phenyl group,
Me: methyl group,
Et: ethyl group,
iPr: isopropyl group,
nPen: n-pentyl group,
cHex: cyclohexyl group,
morp: N-morpholino group,
pipe: N-piperidino group,
SEM: (2-trimethylsilyl)ethoxymethyl group,
Bn: benzyl group,
4-MeO-Bn: 4-methoxybenzyl group,
MOM: methoxymethyl group.

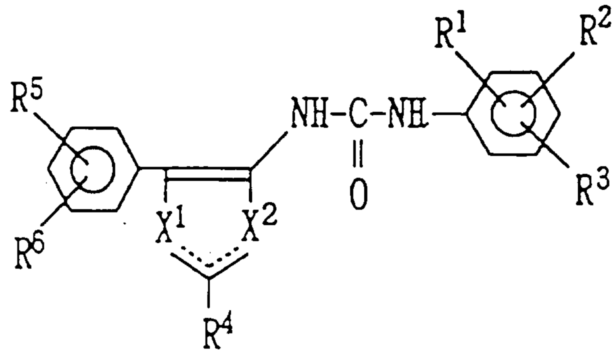

Table 1

| No. | $X^1$ | $X^2$ | $R^1$ | $R^2$ | $R^3$ | $R^4$ | $R^5$ | $R^6$ |
|---|---|---|---|---|---|---|---|---|
| 1 | N | S | 2-iPr | 6-iPr | H | Ph | 2-Cl | H |
| 2 | N | S | 2-iPr | 6-iPr | H | Ph | 2-Me | H |
| 3 | N | S | 2-iPr | 6-iPr | H | Me | H | H |
| 4 | N | S | 2-iPr | 6-iPr | H | Me | 2-Cl | H |
| 5 | N | S | 2-Et | 6-Et | H | Me | 2-Cl | H |
| 6 | N | S | 2-F | 4-F | H | Me | 2-Cl | H |
| 7 | N | S | 2-Et | 6-Et | H | Me | 2-Me | H |
| 8 | N | S | 2-iPr | 6-iPr | H | Me | 2-Me | H |
| 9 | N | S | 2-F | 4-F | H | Me | 2-Me | H |
| 10 | N | S | 2-Et | 6-Et | H | Et | 2-Cl | H |
| 11 | N | S | 2-iPr | 6-iPr | H | Et | 2-Cl | H |
| 12 | N | S | 2-iPr | 6-iPr | H | nPen | 2-Cl | H |
| 13 | N | S | 2-iPr | 6-iPr | H | cHex | 2-Cl | H |
| 14 | N | S | 2-Et | 6-Et | H | $NMe_2$ | 2-Cl | H |
| 15 | N | S | 2-iPr | 6-iPr | H | $NMe_2$ | 2-Cl | H |
| 16 | N | S | 2-F | 4-F | H | $NMe_2$ | 2-Cl | H |
| 17 | N | S | 2-Et | 6-Et | H | pipe | H | H |
| 18 | N | S | 2-iPr | 6-iPr | H | pipe | H | H |
| 19 | N | S | 2-iPr | 6-iPr | H | pipe | 2-Cl | H |
| 20 | N | S | 2-iPr | 6-iPr | H | morp | H | H |
| 21 | N | S | 2-iPr | 6-iPr | H | morp | 2-Cl | H |
| 22 | S | N | 2-Et | 6-Et | H | Ph | 2-Cl | H |

- continued -

Table 1 (cont'd)

| No. | $X^1$ | $X^2$ | $R^1$ | $R^2$ | $R^3$ | $R^4$ | $R^5$ | $R^6$ |
|---|---|---|---|---|---|---|---|---|
| 23 | S | N | 2-iPr | 6-iPr | H | Ph | 2-Cl | H |
| 24 | S | N | 2-iPr | 6-iPr | H | morp | 2-Cl | H |
| 25 | S | N | 2-iPr | 6-iPr | H | Me | 2-Cl | H |
| 26 | S | N | 2-Et | 6-Et | H | Me | 2-Cl | H |
| 27 | S | N | 2-iPr | 6-iPr | H | Ph | 2-Me | H |
| 28 | S | N | 2-Et | 6-Et | H | Ph | 2-Me | H |
| 29 | S | N | 2-iPr | 6-iPr | H | Me | 2-Me | H |
| 30 | S | N | 2-iPr | 6-iPr | H | Ph | 2-SMe | H |
| 31 | O | N | 2-iPr | 6-iPr | H | Ph | 2-Me | H |
| 32 | O | N | 2-iPr | 6-iPr | H | Ph | 2-Cl | H |
| 33 | N | O | 2-iPr | 6-iPr | H | Ph | 2-Me | H |
| 34 | N | N-Me | 2-iPr | 6-iPr | H | Ph | 2-Cl | H |
| 35 | N-Me | N | 2-iPr | 6-iPr | H | Ph | 2-Cl | H |
| 36 | N | N-Bn | 2-iPr | 6-iPr | H | Ph | 2-Cl | H |
| 37 | N-Bn | N | 2-iPr | 6-iPr | H | Ph | 2-Cl | H |
| 38 | N | N-Bn | 2-iPr | 6-iPr | H | Ph | H | H |
| 39 | N | N-SEM | 2-iPr | 6-iPr | H | Ph | 2-Cl | H |
| 40 | N-SEM | N | 2-iPr | 6-iPr | H | Ph | 2-Cl | H |
| 41 | N | N-MOM | 2-iPr | 6-iPr | H | Ph | 2-Cl | H |
| 42 | N-MOM | N | 2-iPr | 6-iPr | H | Ph | 2-Cl | H |
| 43 | N | N-(4-MeO -Bn) | 2-iPr | 6-iPr | H | Ph | 2-Cl | H |

- continued -

Table 1 (cont'd)

| No. | $X^1$ | $X^2$ | $R^1$ | $R^2$ | $R^3$ | $R^4$ | $R^5$ | $R^6$ |
|---|---|---|---|---|---|---|---|---|
| 44 | N-(4-MeO -Bn) | N | 2-iPr | 6-iPr | H | Ph | 2-Cl | H |
| 45 | NH(N) | N(NH) | 2-iPr | 6-iPr | H | Ph | 2-Cl | H |

The azole derivatives of the general formula (I) of the present invention are administered as a prophylactic and therapeutic agent for hypercholesterolemia and atherosclerosis orally or parenterally (intramuscularly, subcutaneously or intravenously). They are administered to human beings preferably orally. Since the azole derivatives of the general formula (I) of the present invention are applicable in themselves as ACAT inhibitors, they are contained in compositions as active ingredients usually in an amount of 0.01 to 100% by weight. Although the dose of the azole derivatives is varied depending on the condition of a disease, age, sex, body weight, administration route, etc., the dose for an adult is usually 0.1 to 1000 mg per day.

When the azole derivative of the general formula (I) of the present invention is formulated into a pharmaceutical form, it is prepared into powder, granules, tablets, dragées, capsules, pills, a suspension, solution, emulsion, ampule, injection, isotonic solution or the like by a conventional preparation method. When an oral solid pharmaceutical is prepared, an excipient and optionally a binder, wetting agent, disintegrator, surfactant, lubricant, dispersant, taste-improver, odor-improver, etc. are added to the active ingredient, and the resulting mixture is made into tablets, coated tablets, granules, capsules or the like by a conventional method. The excipient includes, for example, lactose, glucose, sorbitol, corn starch and mannitol. The binder includes, for example, poly(vinyl alcohol)s, poly(vinyl ether)s, ethyl cellulose, gum arabic, gelatin, hydroxypropyl cellulose and poly(vinylpyrrolidone)s. The disintegrator includes, for example, calcium carbonate, calcium citrate, dextrin, starch and gelatin powder. The lubricant includes, for example, magnesium stearate, talc and poly(ethylene glycol)s. The odor-improver includes, for example, cocoa powder, menthol, and peppermint oil. The tablets and the granules may be properly coated with a frosting, gelatin or the like if necessary. When an injection is prepared, a pH adjustor, buffer, surfactant, solubilizer, solvent, stabilizer, preservative, etc. are added to the active ingredient if necessary, and the resulting mixture is made into a subcutaneous, intramuscular or intravenous injection by a conventional method.

Examples, reference examples, formulation examples and test examples of the present invention are described below but they should not be construed as limiting the scope of the invention.

Example 1

N-[4-(2-Chlorophenyl)-2-phenylthiazol-5-yl]-N'-(2,6-diisopropylphenyl)urea (compound 1)

To a stirred mixture of 1.00 g of 4-(2-chlorophenyl)-2-phenyl-5-thiazolecarboxylic acid and 0.68 cc of diphenylphosphoryl azide in 10 cc of fluorobenzene was added dropwise 0.44 cc of triethylamine at room temperature. The resulting mixture was stirred at room temperature for 10 minutes and then heated at 60 - 70°C for 10 minutes. After cooling, 0.61 cc of 2,6-diisopropylaniline was added, followed by heating at 60 - 70°C for 2 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: ethyl acetate-n-hexane) to obtain 783 mg of compound 1.

Yield 50.5%, m.p. 206 - 207°C.

NMR (δ, ppm ; DMSO-$d_6$) 1.14 (brd, 12H), 3.08 (m, 2H), 7.17 (d, 2H), 7.25-7.70 (m, 8H), 7.85 (d, 2H), 8.12 (s, 1H), 9.12 (s, 1H).

The compound described in Example 2 was obtained in the same manner as in Example 1.

Example 2

N-(2,6-Diisopropylphenyl)-N'-[4-(2-methylphenyl)-2-phenylthiazol-5-yl]urea (compound 2)

Yield 33.8%, m.p. 229 - 231°C.

NMR (δ, ppm ; DMSO-$d_6$) 1.13 (brd, 12H), 2.30 (s, 3H), 3.07 (m, 2H), 7.16 (d, 2H), 7.26 (d, 1H), 7.38-7.47 (m, 7H), 7.85 (d, 2H), 8.16 (s, 1H), 9.00 (s, 1H).

Example 3

N-(2,6-Diisopropylphenyl)-N'-(2-methyl-4-phenylthiazol-5-yl)urea (compound 3)

To a stirred mixture of 658 mg of 2-methyl-4-phenyl-5-thiazolecarboxylic acid and 0.65 cc of diphenylphosphoryl azide in 7 cc of toluene was added dropwise 0.42 cc of triethylamine at room temperature. The resulting mixture was stirred at room temperature for 10 minutes and then heated at 80 - 90°C for 10 minutes. After cooling, 0.57 cc of 2,6-diisopropylaniline was added, followed by heating at 80 - 90°C for 2 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: chloroform) to obtain 873 mg of compound 3.

Yield 73.9%, m.p. 265 - 266°C.

NMR (δ, ppm ; DMSO-$d_6$) 1.13 (d, 12H), 2.55 (s, 3H), 3.11 (m, 2H), 7.15 (d, 2H), 7.25 (t, 1H), 7.37 (t, 1H), 7.50 (t, 2H), 7.75 (d, 2H), 8.11 (s, 1H), 9.04 (s, 1H).

The compounds described in Examples 4 to 13 were obtained in the same manner as in Example 3.

Example 4

N-[4-(2-Chlorophenyl)-2-methylthiazol-5-yl]-N'-(2,6-diisopropylphenyl)urea (compound 4)

Yield 47.7%, m.p. 216 - 219°C.

NMR (δ, ppm ; DMSO-$d_6$) 1.11 (d, 12H), 2.53 (s, 3H), 3.05 (m, 2H), 7.15 (d, 2H), 7.25 (t, 1H), 7.47-7.54 (m, 3H), 7.64 (t, 1H), 7.98 (s, 1H), 8.82 (s, 1H).

Example 5

N-[4-(2-Chlorophenyl)-2-methylthiazol-5-yl]-N'-(2,6-diethylphenyl)urea (compound 5)

Yield 62.6%, m.p. 204 - 206°C.

NMR (δ, ppm ; DMSO-$d_6$) 1.09 (t, 6H), 2.48-2.53 (m, 7H), 7.09 (d, 2H), 7.17 (t, 1H), 7.47-7.52 (m, 3H), 7.63 (d, 1H), 8.02 (s, 1H), 8.83 (s, 1H).

Example 6

N-[4-(2-Chlorophenyl)-2-methylthiazol-5-yl]-N'-(2,4-difluorophenyl)urea (compound 6)

Yield 59.9%, amorphous.

NMR (δ, ppm ; DMSO-$d_6$) 2.57 (s, 3H), 7.05 (m, 1H), 7.31 (m, 1H), 7.47-7.51 (m, 3H), 8.11 (m, 1H), 8.97 (s, 1H), 9.16 (s, 1H).

Example 7

N-(2,6-Diethylphenyl)-N'-[2-methyl-4-(2-methylphenyl)thiazol-5-yl]urea (compound 7)

Yield 60.8%, m.p. 215 - 216°C.

NMR (δ, ppm ; DMSO-$d_6$) 1.08 (t, 6H), 2.23 (s, 3H), 2.47-2.53 (m, 7H), 7.07-7.18 (m, 3H), 7.32-7.36 (m, 4H), 8.05 (s, 1H), 8.69 (s, 1H).

Example 8

N-(2,6-Diisopropylphenyl)-N'-[2-methyl-4-(2-methylphenyl)thiazol-5-yl]urea (compound 8)

Yield 64.0%, m.p. 229.5 - 230.5°C.

NMR (δ, ppm ; DMSO-$d_6$) 1.11 (brd, 12H), 2.24 (s, 3H), 2.53 (s, 3H), 3.04 (m, 2H), 7.14 (d, 2H), 7.24 (t, 1H), 7.33-7.37 (m, 4H), 8.01 (s, 1H), 8.68 (s, 1H).

Example 9

N-(2,4-Difluorophenyl)-N'-[2-methyl-4-(2-methylphenyl)thiazol-5-yl]urea (compound 9)

Yield 61.6%, amorphous.

NMR (δ, ppm ; DMSO-$d_6$) 2.18 (s, 3H), 2.56 (s, 3H), 7.05 (m, 1H), 7.30-7.34 (m, 5H), 8.11 (m, 1H), 9.01 (s, 1H), 9.06 (s, 1H).

Example 10

N-[4-(2-Chlorophenyl)-2-ethylthiazol-5-yl]-N'-(2,6-diethylphenyl)urea (compound 10)

Yield 68.7%, m.p. 205 - 207°C.

NMR (δ, ppm ; DMSO-$d_6$) 1.08 (t, 6H), 1.27 (t, 3H), 2.46-2.53 (m, 4H), 2.87 (q, 2H), 7.08-7.19 (m, 3H), 7.46-7.63 (m, 4H), 8.01 (s, 1H).

Example 11

N-[4-(2-Chlorophenyl)-2-ethylthiazol-5-yl]-N'-(2,6-diisopropylphenyl)urea (compound 11)

Yield 65.2%, m.p. 183 - 185°C.

NMR (δ, ppm ; DMSO-$d_6$) 1.11 (d, 12H), 1.27 (t, 3H), 2.86 (q, 2H), 3.06 (m, 2H), 7.14 (d, 2H), 7.23-7.25 (m, 1H), 7.47-7.65 (m, 4H), 7.98 (s, 1H), 8.81 (s, 1H).

Example 12

N-[4-(2-Chlorophenyl)-2-n-pentylthiazol-5-yl]-N'-(2,6-diisopropylphenyl)urea (compound 12)

Yield 66.5%, m.p. 169 - 171°C.

NMR (δ, ppm ; DMSO-$d_6$) 0.87 (t, 3H), 1.11 (brd, 12H), 1.29-1.35 (m, 4H), 1.67-1.71 (m, 2H), 2.81-2.85 (m, 2H), 3.05 (m, 2H), 7.15 (d, 2H), 7.24 (m, 1H), 7.47-7.65 (m, 4H), 7.99 (s, 1H), 8.83 (s, 1H).

Example 13

N-[4-(2-Chlorophenyl)-2-cyclohexylthiazol-5-yl]-N'-(2,6-diisopropylphenyl)urea (compound 13)

Yield 69.3%, m.p. 235 - 236°C.

NMR (δ, ppm ; DMSO-$d_6$) 1.11 (brd, 12H), 1.21-1.67 (m, 6H), 1.74-1.78 (m, 2H), 2.00-2.09 (m, 2H), 2.82-2.88 (m, 1H), 3.05 (m, 2H), 7.14 (d, 2H), 7.25 (t, 1H), 7.47-7.54 (m, 3H), 7.62-7.65 (m, 1H), 7.89 (s, 1H), 8.81 (s, 1H).

Example 14

N-[4-(2-Chlorophenyl)-2-dimethylaminothiazol-5-yl]-N'-(2,6-diethylphenyl)urea (compound 14)

To a stirred mixture of 566 mg of 4-(2-chlorophenyl)-2-dimethylamino-5-thiazolecarboxylic acid and 0.44 cc of diphenylphosphoryl azide in 6 cc of fluorobenzene was added dropwise 0.28 cc of triethylamine at room temperature. The resulting mixture was stirred at room temperature for 10 minutes and refluxed for 15 minutes. After cooling, 0.33 cc of 2,6-diethylaniline was added, followed by refluxing for 1.5 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: chloroform) to obtain 642 mg of compound 14.

Yield 74.9%, m.p. 244 - 246°C.

NMR (δ, ppm ; DMSO-$d_6$) 1.07 (t, 6H), 2.47 (q, 4H), 2.97 (s, 6H), 7.06 (d, 2H), 7.14 (t, 1H), 7.40-7.55 (m, 4H), 7.77 (s, 1H), 8.26 (s, 1H).

The compounds described in Examples 15 to 21 were obtained in the same manner as in Example 14.

Example 15

N-[4-(2-Chlorophenyl)-2-dimethylaminothiazol-5-yl]-N'-(2,6-diisopropylphenyl)urea (compound 15)

Yield 80.5%, m.p. 241 - 242°C.

NMR (δ, ppm ; DMSO-$d_6$) 1.11 (d, 12H), 2.98 (s, 6H), 3.04 (m, 2H), 7.13 (d, 2H), 7.23 (t, 1H), 7.42-7.59 (m, 4H), 7.75 (s, 1H), 8.25 (s, 1H).

Example 16

N-[4-(2-Chlorophenyl)-2-dimethylaminothiazol-5-yl]-N'-(2,4-difluorophenyl)urea (compound 16)

Yield 71.1%, m.p. 215 - 217°C.

NMR (δ, ppm ; DMSO-$d_6$) 2.98 (s, 6H), 7.02-7.04 (m, 1H), 7.27-7.30 (m, 1H), 7.42-7.47 (m, 3H), 7.56-7.58 (m, 1H), 8.07-8.08 (m, 1H), 8.67 (s, 1H), 8.76 (s, 1H).

Example 17

N-(2,6-Diethylphenyl)-N'-[4-phenyl-2-(N-piperidino)thiazol-5-yl)urea (compound 17)

Yield 42.2%, m.p. 253 - 254°C.

NMR (δ, ppm ; DMSO-$d_6$) 1.10 (t, 6H), 1.60 (m, 6H), 2.50 (m, 4H), 3.40 (m, 4H), 7.07 (d, 2H), 7.15 (t, 1H), 7.29-7.43 (m, 3H), 7.81-7.86 (m, 3H), 8.40 (s, 1H).

Example 18

N-(2,6-Diisopropylphenyl)-N'-[4-phenyl-2-(N-piperidino)thiazol-5-yl)urea (compound 18)

Yield 40.3%, m.p. 245 - 248°C.

NMR (δ, ppm ; DMSO-$d_6$) 1.11 (d, 12H), 1.60 (m, 6H), 3.11 (m, 2H), 3.40 (m, 4H), 7.12 (d, 2H), 7.22 (t, 1H), 7.32-7.44 (m, 3H), 7.81-7.83 (m, 3H), 8.37 (s, 1H).

Example 19

N-[4-(2-Chlorophenyl)-2-(N-piperidino)thiazol-5-yl]-N'-(2,6-diisopropylphenyl)urea (compound 19)

Yield 70.4%, m.p. 248 - 250°C.

NMR (δ, ppm ; DMSO-$d_6$) 1.09 (d, 12H), 1.58 (m, 6H), 3.04 (m, 2H), 3.32 (m, 4H), 7.12 (d, 2H), 7.22 (t, 2H), 7.41-7.57 (m, 4H), 7.75 (s, 1H), 8.28 (s, 1H).

Example 20

N-(2,6-Diisopropylphenyl)-N'-[2-(N-morpholino)-4-phenylthiazol-5-yl)urea (compound 20)

Yield 29.3%, m.p. 254 - 257°C.

NMR (δ, ppm ; DMSO-$d_6$) 1.11 (d, 12H), 3.11 (m, 2H), 3.36 (m, 4H), 3.71 (m, 4H), 7.12 (d, 2H), 7.22 (t, 1H), 7.33-7.45 (m, 3H), 7.80-7.82 (m, 2H), 7.87 (s, 1H), 8.49 (s, 1H).

Example 21

N-[4-(2-Chlorophenyl)-2-(N-morpholino)thiazol-5-yl]-N'-(2,6-diisopropylphenyl)urea (compound 21)

Yield 73.9%, m.p. 258 - 261°C.

NMR (δ, ppm ; DMSO-$d_6$) 1.10 (d, 12H), 3.04 (m, 2H), 3.30 (m, 4H), 3.70 (m, 4H), 7.13 (d, 2H), 7.23 (t, 1H), 7.43 (t, 2H), 7.51 (t, 1H), 7.59 (t,1H), 7.80 (s, 1H), 8.40 (s, 1H).

Example 22

N-[5-(2-Chlorophenyl)-2-phenylthiazol-4-yl]-N'-(2,6-diethylphenyl)urea (compound 22)

A mixture of 500 mg of 4-amino-5-(2-chlorophenyl)-2-phenylthiazole and 315 mg of 2,6-diethylphenyl isocyanate in 10 cc of toluene was refluxed for 10 hours. After cooling, the solvent was distilled off under reduced pressure, and the crude product thus obtained was purified by a silica gel column chromatography (eluent: ethyl acetate-chloroform) to obtain 311 mg of compound 22.

Yield 38.6%, m.p. 207 - 208°C.

NMR (δ, ppm ; DMSO-$d_6$) 1.07 (t, 6H), 2.45 (q, 4H), 7.04 (d, 2H), 7.12 (t, 1H), 7.38-7.44 (m, 2H), 7.52-7.75 (m, 5H), 7.93-7.97 (m, 3H), 8.85 (s, 1H).

The compound described in Example 23 was obtained in the same manner as in Example 22.

Example 23

N-[5-(2-Chlorophenyl)-2-phenylthiazol-4-yl]-N'-(2,6-diisopropylphenyl)urea (compound 23)

Yield 37.7%, m.p. 188 - 189°C.

NMR (δ, ppm ; DMSO-$d_6$) 1.07 (brd, 12H), 3.07 (m, 2H), 7.09 (d, 2H), 7.18 (t, 1H), 7.39-7.42 (m, 2H), 7.52-7.57 (m, 5H), 7.92-7.96 (m, 3H), 8.82 (s, 1H).

Example 24

N-[5-(2-Chlorophenyl)-2-(N-morpholino)thiazol-4-yl]-N'-(2,6-diisopropylphenyl)urea (compound 24)

To a stirred mixture of 450 mg of 5-(2-chlorophenyl)-2-(N-morpholino)-4-thiazolecarboxylic acid and 0.30 cc of diphenylphosphoryl azide in 5 cc of fluorobenzene was added dropwise 0.20 cc of triethylamine at room temperature. The resulting mixture was stirred at room temperature for 10 minutes and then heated at 60 - 70°C for 5 minutes. After cooling, 0.27 cc of 2,6-diisopropylaniline was added, followed by heating at 60 - 70°C for 2 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: ethyl acetate-n-hexane) to obtain 550 mg of compound 24.

Yield 79.5%, m.p. 142 - 144°C.

NMR (δ, ppm ; DMSO-$d_6$) 1.08 (brd, 12H), 3.08 (m, 2H), 3.40 (t, 4H), 3.73 (t, 4H), 7.09 (d, 2H), 7.20 (t, 1H), 7.30-7.34 (m, 2H), 7.44-7.48 (m, 2H), 8.08 (s, 1H), 8.40 (s, 1H).

Example 25

N-[5-(2-Chlorophenyl)-2-methylthiazol-4-yl]-N'-(2,6-diisopropylphenyl)urea (compound 25)

To a stirred mixture of 3.00 g of 5-(2-chlorophenyl)-2-methyl-4-thiazolecarboxylic acid and 2.80 cc of diphenylphosphoryl azide in 30 cc of fluorobenzene was added dropwise 1.81 cc of triethylamine at room temperature. The resulting mixture was stirred at room temperature for 10 minutes and then heated at 55 - 60°C for 5 minutes. After cooling, 2.45 cc of 2,6-diisopropylaniline was added, followed by heating at 60 - 70°C for 3 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: ethyl acetate-n-hexane) to obtain 3.41 g of compound 25.

Yield 67.4%, m.p. 177 - 180°C.

NMR (δ, ppm ; $CDCl_3$) 1.25 (d, 12H), 2.70 (s, 3H), 3.24 (m, 2H), 6.55 (s, 1H), 7.21 (d, 2H), 7.29 (t, 1H), 7.34-7.39 (m, 2H), 7.44-7.47 (m, 1H), 7.51-7.54 (m, 1H), 9.91 (s, 1H).

The compounds described in Examples 26 to 29 were obtained in the same manner as in Example 25.

Example 26

N-[5-(2-Chlorophenyl)-2-methylthiazol-4-yl]-N'-(2,6-diethylphenyl)urea (compound 26)

Yield 65.1%, m.p. 187 - 188°C.

NMR (δ, ppm ; $CDCl_3$) 1.24 (t, 6H), 2.70 (s, 3H), 2.71 (q, 4H), 6.53 (s, 1H), 7.15 (d, 2H), 7.22 (dd, 1H), 7.33-7.39 (m, 2H), 7.41-7.45 (m, 1H), 7.51-7.54 (m, 1H), 9.96 (s, 1H).

Example 27

N-(2,6-Diisopropylphenyl)-N'-[5-(2-methylphenyl)-2-phenylthiazol-4-yl]urea (compound 27)

Yield 64.2%, m.p. 191 - 193°C.

NMR (δ, ppm ; DMSO-$d_6$) 1.07 (d, 12H), 2.32 (s, 3H), 3.04 (m, 2H), 7.08-7.27 (m, 4H), 7.32-7.38 (m, 3H), 7.50-7.55 (m, 3H), 7.90 (s, 1H), 7.94 (d, 2H), 8.50 (s, 1H).

Example 28

N-(2,6-Diethylphenyl)-N'-[5-(2-methylphenyl)-2-phenylthiazol-4-yl]urea (compound 28)

Yield 74.5%, m.p. 212 - 213°C.

NMR (δ, ppm ; DMSO-$d_6$) 1.05 (t, 6H), 2.31 (s, 3H), 2.43 (q, 4H), 7.02-7.11 (m, 3H), 7.24-7.38 (m, 4H), 7.50-7.53 (m, 3H), 7.93-7.95 (m, 3H), 8.54 (s, 1H).

Example 29

N-(2,6-Diisopropylphenyl)-N'-[2-methyl-5-(2-methylphenyl)thiazol-4-yl]urea (compound 29)

Yield 47.3%, m.p. 132 - 134°C.

NMR (δ, ppm ; DMSO-$d_6$) 1.06 (d, 12H), 2.65 (s, 3H), 2.97-3.01 (m, 2H), 7.07-7.09 (m, 2H), 7.16-7.29 (m, 5H), 7.89 (brs, 1H), 8.25 (brs, 1H).

Example 30

N-(2,6-Diisopropylphenyl)-N'-[5-(2-methylthiophenyl)-2-phenylthiazol-4-yl]urea (compound 30)

A mixture of 3.00 g of 4-amino-5-(2-methylthiophenyl)-2-phenylthiazole and 2.20 ml of 2,6-diisopropylpheny isocyanate in 30 cc of ethyl acetate was refluxed for 16 hours. After cooling, the solvent was distilled off under reduced pressure, and the crude product thus obtained was purified by a silica gel column chromatography (eluent: ethyl acetate-chloroform) to obtain 4.02 g of compound 30.

Yield 82.3%, m.p. 151 - 153°C.

NMR (δ, ppm ; $CDCl_3$) 1.25 (d, 12H), 2.48 (s, 3H), 3.32 (m, 2H), 6.63 (s, 1H), 7.22-7.34 (m, 5H), 7.37-7.46 (m, 5H), 7.84-7.87 (m, 2H), 9.92 (s, 1H).

Example 31

N-(2,6-Diisopropylphenyl)-N'-[5-(2-methylphenyl)-2-phenyloxazol-4-yl]urea (compound 31)

To a stirred mixture of 600 mg of 5-(2-methylphenyl)-2-phenyl-4-oxazolecarboxylic acid and 0.48 cc of diphenylphosphoryl azide in 10 cc of fluorobenzene was added dropwise 0.30 cc of triethylamine at room temperature. The resulting mixture was stirred at room temperature for 10 minutes and then heated at 50 - 55°C for 5 minutes. After cooling, 0.42 cc of 2,6-diisopropylaniline was added, followed by heating at 60 - 70°C for 3 hours. After cooling, water was added to the reaction mixture, followed by extraction with chloroform. The extract was dried over magnesium sulfate and then distilled under reduced pressure to remove the solvent. The crude product thus obtained was purified by a silica gel column chromatography (eluent: ethyl acetate-n-hexane) to obtain 584 mg of compound 31.

Yield 59.9%, m.p. 237 - 239°C.

NMR (δ, ppm ; $CDCl_3$) 1.28 (d, 12H), 2.48 (s, 3H), 3.32 (m, 2H), 6.53 (s, 1H), 7.23 (d, 2H), 7.30-7.36 (m, 4H), 7.45-7.48 (m, 4H), 7.98-8.01 (m, 2H), 9.47 (s, 1H).

The compounds described in Examples 32 and 33 were obtained in the same manner as in Example 31.

Example 32

N-[5-(2-Chlorophenyl)-2-phenyloxazol-4-yl]-N'-(2,6-diisopropylphenyl)-N'-urea (compound 32)

Yield 74.5%, m.p. 222 - 224°C.

NMR (δ, ppm ; $CDCl_3$) 1.22 (d, 12H), 3.30 (m, 2H), 6.69 (s, 1H), 7.23 (d, 2H), 7.38-7.61 (m, 8H), 8.00-8.05 (m, 2H), 9.44 (s, 1H).

Example 33

N-(2,6-Diisopropylphenyl)-N'-[4-(2-methylphenyl)-2-phenyloxazol-5-yl]urea (compound 33)

Yield 75.4%, m.p. 257 - 259°C.

NMR (δ, ppm ; DMSO-$d_6$) 1.09 (d, 12H), 2.49 (s, 3H), 3.08 (m, 2H), 7.11 (d, 2H), 7.20-7.26 (m, 2H), 7.33 (d, 2H), 7.49-7.58 (m, 4H), 7.99 (d, 2H), 8.05 (s, 1H), 8.67 (s, 1H).

Example 34

N-[4-(2-Chlorophenyl)-1-methyl-2-phenylimidazol-5-yl]-N'-(2,6-diisopropylphenyl)urea (compound 34)

To a stirred mixture of 219 mg of 4-(2-chlorophenyl)-1-methyl-2-phenyl-5-imidazolecarboxylic acid and 0.16 cc of diphenylphosphoryl azide in 3 cc of fluorobenzene was added dropwise 0.10 cc of triethylamine at room temperature. The resulting mixture was stirred at room temperature for 10 minutes and then heated at 60 - 70°C for 15 minutes. After cooling, 0.14 cc of 2,6-diisopropylaniline was added, followed by heating at 60 - 70°C for 4 hours. After cooling, water was added to the reaction mixture, followed by extraction with chloroform. The extract was dried over magnesium sulfate and then distilled under reduced pressure to remove the solvent. The crude product thus obtained was purified by

a silica gel column chromatography (eluent: methanol-chloroform) to obtain 242 mg of compound 33.

Yield 71.0%, m.p. 226 - 228°C.

NMR (δ, ppm ; DMSO-$d_6$) 1.11 (brd, 12H), 3.15 (m, 2H), 6.62 (s, 3H), 7.11-7.22 (m, 3H), 7.37-7.56 (m, 7H), 7.76 (d, 2H), 7.87 (brs, 1H), 8.14 (s, 1H).

The compounds described in Examples 35 to 44 were obtained in the same manner as in Example 34.

Example 35

N-[5-(2-Chlorophenyl)-1-methyl-2-phenylimidazol-4-yl]-N'-(2,6-diisopropylphenyl)urea (compound 35)

Yield 61.5%, m.p. 135 - 137°C.

NMR (δ, ppm ; DMSO-$d_6$) 1.08 (d, 12H), 3.08-3.10 (m, 2H), 3.48 (s, 3H), 7.09-7.19 (m, 3H), 7.45-7.63 (m, 7H), 7.74 (d, 2H), 8.12 (s, 1H), 8.38 (brs, 1H).

Example 36

N-[1-Benzyl-4-(2-chlorophenyl)-2-phenylimidazol-5-yl]-N'-(2,6-diisopropylphenyl)urea (compound 36)

Yield 81.7%, m.p. 182 - 185°C.

NMR (δ, ppm ; DMSO-$d_6$) 1.02 (brd, 12H), 2.96 (m, 2H), 5.32 (s, 2H), 7.06-7.41 (m, 13H), 7.54-7.56 (m, 4H), 7.73 (brs, 1H), 8.04 (brs, 1H).

Example 37

N-[1-Benzyl-5-(2-chlorophenyl)-2-phenylimidazol-4-yl]-N'-(2,6-diisopropylphenyl)urea (compound 37)

Yield 62.2%, m.p. 218 - 220°C.

NMR (δ, ppm ; DMSO-$d_6$) 1.07 (d, 12H), 3.08 (m, 2H), 5.07 (d, 1H), 5.28 (d, 1H), 6.71 (d, 2H), 7.09-7.50 (m, 13H), 7.61-7.63 (m, 2H), 8.16 (s, 1H), 8.41 (brs, 1H).

Example 38

N-[1-Benzy1-2,4-diphenylimidazol-5-yl]-N'-(2,6-diisopropylphenyl)urea (compound 38)

Yield 54.4%, m.p. 212 - 214°C.

NMR (δ, ppm ; DMSO-$d_6$) 1.09 (m, 12H), 3.16 (m, 2H), 5.27 (brd, 2H), 7.10-7.42 (m, 14H), 7.57 (m, 2H), 7.96 (m, 3H), 8.19 (m, 1H).

Example 39

N-[4-(2-Chlorophenyl)-2-phenyl-1-(2-trimethylsilyl)ethoxymethylimidazol-5-yl]-N'-(2,6-diisopropylphenyl)urea (compound 39)

Yield 67.0%, m.p. 192 - 195°C.

NMR (δ, ppm ; DMSO-$d_6$ 0.03 (s, 9H), 0.94 (t, 2H), 1.09 (brd, 12H), 3.09 (m, 2H), 3.68 (t, 2H), 5.27 (s, 2H), 7.10-7.21 (m, 3H), 7.38-7.57 (m, 7H), 7.99 (brd, 3H), 8.19 (s, 1H).

Example 40

N-[5-(2-Chlorophenyl)-2-phenyl-1-(2-trimethylsilyl)ethoxymethylimidazol-4-yl]-N'-(2,6-diisopropylphenyl)urea (compound 40)

Yield 48.7%, m.p. 165 - 167°C.

NMR (δ, ppm ; DMSO-$d_6$) 0.13 (s, 9H), 0.65 (t, 2H), 1.07 (d, 12H), 3.11 (m, 4H), 5.04 (d, 1H), 5.26 (d, 1H), 7.09-7.19 (m, 3H), 7.44-7.62 (m, 7H), 7.82 (d, 2H), 8.20 (s, 1H), 8.31 (brs, 1H).

Example 41

N-[4-(2-Chlorophenyl)-1-methoxymethyl-2-phenylimidazol-5-yl]-N'-(2,6-diisopropylphenyl)urea (compound 41)

Yield 51.9%, m.p. 189 - 192°C.

NMR (δ, ppm ; DMSO-$d_6$) 1.08 (brd, 12H), 3.07 (m, 2H), 3.43 (s, 3H), 5.23 (s, 2H), 7.09-7.21 (m, 3H), 7.37-7.56 (m, 7H), 7.83-7.98 (m, 3H), 8.20 (s, 1H).

Example 42

N-[5-(2-Chlorophenyl)-1-methoxymethyl-2-phenylimidazol-4-yl]-N'-(2,6-diisopropylphenyl)urea (compound 42)

Yield 68.8%, m.p. 175 - 177°C.

NMR (δ, ppm ; DMSO-$d_6$) 1.07 (d, 12H), 3.00 (s, 3H), 3.08 (m, 2H), 4.93 (d, 1H), 5.20 (d, 1H), 7.08-7.19 (m, 3H), 7.45-7.62 (m, 9H), 7.79 (d, 2H), 8.19 (s, 1H), 8.27 (brs, 1H).

Example 43

N-[4-(2-Chlorophenyl)-1-(4-methoxybenzyl)-2-phenylimidazol-5-yl]-N'-(2,6-diisopropylphenyl)urea (compound 43)

Yield 76.1%, m.p. 194 - 197°C.

NMR (δ, ppm ; DMSO-$d_6$) 1.03 (brd, 12H), 2.97 (m, 2H), 3.71 (s, 3H), 5.24 (s, 2H), 6.88 (d, 2H), 7.01 (d, 2H), 7.07-7.19 (m, 3H), 7.36-7.42 (m, 5H), 7.53-7.58 (m, 4H), 7.74 (brs, 1H), 8.03 (brs, 1H).

Example 44

N-[5-(2-Chlorophenyl)-1-(4-methoxybenzyl)-2-phenylimidazol-4-yl]-N'-(2,6-diisopropylphenyl)urea (compound 44)

Yield 45.6%, m.p. 197 - 198°C.

NMR (δ, ppm ; DMSO-$d_6$) 1.07 (d, 12H), 3.08 (m, 2H), 3.64 (s, 3H), 4.99 (d, 1H), 5.21 (d, 1H), 6.60-6.62 (m, 2H), 6.70 (d, 2H), 7.10 (d, 2H), 7.17-7.19 (m, 1H), 7.32-7.53 (m, 7H), 7.63 (d, 2H), 8.14 (s, 1H), 8.42 (brs, 1H).

Example 45

N-[4(5)-(2-Chlorophenyl)-2-phenylimidazol-5(4)-yl]-N'-(2,6-diisopropylphenyl)urea (compound 45)

To a stirred solution of 211 mg of N-[5-(2-chlorophenyl)-2-phenyl-1-(2-trimethylsilylethoxy)methylimidazol-4-yl]-N'-(2,6-diisopropylphenyl)urea in 3 cc of tetrahydrofuran (THF) was added dropwise 0.70 cc of tetra-n-butylammonium fluoride (1M solution/THF) under ice-cooling. The resulting solution was refluxed for 6 hours and then distilled to remove the solvent, followed by extraction with chloroform. The organic layer was washed with water and dried over magnesium sulfate. The solvent was distilled off under reduced pressure and the crude product thus obtained was purified by a silica gel column chromatography (eluent: ethyl acetate-n-hexane) to obtain 39.7 mg of compound 44.

Yield 24.0%, m.p. 147 - 152°C.

NMR (δ, ppm ; DMSO-$d_6$) 1.10 (d, 12H), 3.15 (m, 2H), 7.10-7.20 (m, 3H), 7.36-7.59 (m, 7H), 7.97 (d, 2H), 8.20 (s, 1H), 8.38 (brs, 1H), 12.70 (brs, 1H).

Reference Example 1

4-(2-Chlorophenyl)-2-phenyl-5-thiazolecarboxylic acid

1) Ethyl 4-(2-chlorophenyl)-2-phenyl-5-thiazolecarboxylate (compound A)

A mixture of 4.60 g of ethyl 2-chloro-3-(2-chlorophenyl)-3-oxopropionate and 2.41 g of benzthioamide in 20 cc of ethanol was heated under reflux for 6 hours. After cooling, the solvent was distilled off under reduced pressure and ethyl acetate was added to the residue. The organic layer was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 1/9) to obtain 5.60 g of compound A. Yield: 92.5%.

2) 4-(2-Chlorophenyl)-2-phenyl-5-thiazolecarboxylic acid

A mixture of 6.1 g of compound A and 2.10 g of potassium hydroxide in 20 cc of ethanol and 2 cc of water was heated under reflux for 5 hours. After cooling, the solvent was distilled off under reduced pressure and water was added to the residue. The aqueous layer was acidified with 1N HCl, and extracted with ethyl acetate. The extract was dried over magnesium sulfate and then distilled to remove the solvent. The crystals thus obtained were filtered and washed with n-hexane to obtain 5.17 g of 4-(2-chlorophenyl)-2-phenyl-5-thiazolecarboxylic acid. Yield: 92.3%.

Reference Example 2

2-Methyl-4-phenyl-5-thiazolecarboxylic acid

1) Ethyl 2-methyl-4-phenyl-5-thiazolecarboxylate (compound B)

A mixture of 6.80 g of ethyl 2-chloro-3-phenyl-3-oxopropionate and 2.25 g of acetothioamide in 75 cc of ethanol was heated under reflux for 6 hours. After cooling, the solvent was distilled off under reduced pressure and ethyl acetate was added to the residue. The organic layer was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 1/4) to obtain 4.00 g of compound B. Yield: 53.9%.

2) 4-Phenyl-2-methyl-5-thiazolecarboxylic acid

A mixture of 4.00 g of compound B and 1.81 g of potassium hydroxide in 40 cc of ethanol and 10 cc of water was heated under reflux for 3 hours. After cooling, the solvent was distilled off under reduced pressure and ice water was added to the residue, followed by washing with n-hexane. The aqueous layer was acidified with 1N HCl and the crystals thus precipitated were collected by filtration, washed with water, and then dried to obtain 3.53 g of 4-phenyl-2-methyl-5-thiazolecarboxylic acid. Yield: 99.3%.

Reference Example 3

4-(2-Chlorophenyl)-2-(N-piperidino)-5-thiazolecarboxylic acid

1) Ethyl 4-(2-chlorophenyl)-2-(N-piperidino)-5-thiazolecarboxylate (compound C)

A mixture of 1.04 g of ethyl 2-chloro-3-(2-chlorophenyl)-3-oxopropionate and 0.58 g of 1-thiocarbamoylpiperidine in 10 cc of ethanol was heated under reflux for 3 hours. After cooling, the solvent was distilled off under reduced pressure and ethyl acetate was added to the residue. The organic layer was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 1/4) to obtain 0.99 g of compound C. Yield: 70.5%.

2) 4-(2-Chlorophenyl)-2-(N-piperidino)-5-thiazolecarboxylic acid

A mixture of 1.89 g of compound C and 0.60 g of potassium hydroxide in 15 cc of ethanol and 4 cc of water was heated under reflux for 3 hours. After cooling, the solvent was distilled off under reduced pressure and ice water was added to the residue, followed by washing with n-hexane. The aqueous layer was acidified with 1N HCl and extracted with chloroform. The extract was dried over magnesium sulfate and then distilled to remove the solvent. The crystals thus obtained were filtered and washed with n-hexane to obtain 1.39 g of 4-(2-chlorophenyl)-2-(N-piperidino)-5-thiazolecarboxylic acid. Yield: 79.7%.

The following compounds were obtained in the same manner as in Reference Examples 1,2 and 3:

(1) 4-(2-methylphenyl)-2-phenyl-5-thiazolecarboxylic acid,
(2) 4-(2-chlorophenyl)-2-methyl-5-thiazolecarboxylic acid,
(3) 2-methyl-4-(2-methylphenyl)-5-thiazolecarboxylic acid,
(4) 4-(2-chlorophenyl)-2-ethyl-5-thiazolecarboxylic acid,
(5) 4-(2-chlorophenyl)-2-pentyl-5-thiazolecarboxylic acid,
(6) 4-(2-chlorophenyl)-2-cyclohexyl-5-thiazolecarboxylic acid,
(7) 2-(N-morpholino)-4-phenyl-5-thiazolecarboxylic acid,
(8) 4-(2-chlorophenyl)-2-(N-morpholino)-5-thiazolecarboxylic acid.

Reference Example 4

4-(2-Chlorophenyl)-2-dimethylamino-5-thiazolecarboxylic acid

1) N-(2-Chlorobenzoyl)-N',N'-dimethylthiourea (compound D)

To a stirred solution of 8.37 g of ammonium thiocyanate in 50 cc of acetone was added dropwise 17.50 g of 2-chlorobenzoyl chloride at room temperature, and the mixture was heated under reflux for 5 minutes. A mixture of 9.02 g of a 50% aqueous dimethylamine solution and 10 cc of acetone was added dropwise with refluxing, and the resulting mixture was refluxed for 3.5 hours. The reaction mixture was poured into water 10 times as much as the reaction mixture with stirring, and the crystals thus precipitated were collected by filtration and washed with water. The crystals were dissolved in chloroform and washed with a saturated aqueous sodium chloride solution. The organic layer was dried over magnesium sulfate and then distilled under reduced pressure to remove the solvent, whereby 8.81 g of compound D was obtained. Yield: 36.3%.

2) Ethyl 4-(2-chlorophenyl)-2-dimethylamino-5-thiazolecarboxylate (compound E)

A mixture of 8.50 g of compound D and 5.85 g of ethyl bromoacetate in 50 cc of ethanol was heated under reflux for 10 minutes. To the mixture was added 5.00 ml of triethylamine, followed by heating under reflux for 13 hours. After cooling, water was added to the reaction mixture, followed by three runs of extraction with ethyl acetate. The combined organic layer was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 1/4) to obtain 6.18 g of compound E. Yield: 56.8%.

3) 4-(2-Chlorophenyl)-2-dimethylamino-5-thiazolecarboxylic acid

A mixture of 5.60 g of compound E and 2.02 g of potassium hydroxide in 25 cc of ethanol and 5 cc of water was heated under reflux for 5 hours. After cooling, the solvent was distilled off under reduced pressure and ice water was added to the residue, followed by washing with n-hexane. The aqueous layer was acidified with 1N HCl and extracted with chloroform. The extract was dried over magnesium sulfate and then distilled to remove the solvent. The crystals thus obtained were filtered and washed with n-hexane to obtain 4.84 g of 4-(2-chlorophenyl)-2-dimethylamino-5-thiazolecarboxylic acid. Yield: 95.1%.

The following compound was obtained in the same manner as in Reference Example 4:
4-phenyl-2-(N-piperidino)-5-thiazolecarboxylic acid.

Reference Example 5

4-Amino-5-(2-chlorophenyl)-2-phenylthiazole

A mixture of 3.00 g of 2-chloro-2-(2-chlorophenyl)acetonitrile and 2.21 g of benzthioamide in 6 cc of ethanol was

heated under reflux for 6 hours. The mixture was poured into an aqueous sodium hydrogencarbonate solution, followed by extraction with chloroform. The extract was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 1/4) to obtain 1.72 g of 4-amino-5-(2-chlorophenyl)-2-phenylthiazole. Yield: 37.3%.

The following compound was obtained in the same manner as in Reference Example 5:
4-amino-5-(2-methylthiophenyl)-2-phenylthiazole.

Reference Example 6

5-(2-Chlorophenyl)-2-methyl-4-thiazolecarboxylic acid

1) Ethyl 5-(2-chlorophenyl)-2-methyl-4-thiazolecarboxylate (compound F)

To a stirred solution of 11.50 g of ethyl 3-(2-chlorophenyl)-2-oxopropionate in 70 ml of carbon disulfide was added dropwise a solution of 2.62 ml of bromine in 10 ml of carbon disulfide under ice-cooling. The resulting solution was stirred at room temperature for 30 minutes and then poured into a mixed aqueous solution of sodium hydrogencarbonate and sodium thiosulfate, followed by extraction with diethyl ether. The extract was washed with a saturated aqueous sodium chloride solution and dried over magnesium sulfate. The solvent was distilled off to obtain a crude extract of ethyl 3-bromo-3-(2-chlorophenyl)-2-oxopropionate. To the crude extract were added 3.82 g of thioacetamide and 75 cc of ethanol and the resulting mixture was heated under reflux for 5 hours. After cooling, the solvent was distilled off under reduced pressure and an aqueous sodium hydrogencarbonate solution was added to the residue, followed by extraction with chloroform. The extract was washed with a saturated aqueous sodium chloride solution and dried over magnesium sulfate. The solvent was distilled off under reduced pressure and the crude product thus obtained was purified by a silica gel column chromatography (eluent: ethyl acetate/n-hexane = 2/3) to obtain 10.17 g of compound F. Yield: 71.1%.

2) 5-(2-Chlorophenyl)-2-methyl-4-thiazolecarboxylic acid

A mixture of 10.17 g of compound F and 2.56 g of potassium hydroxide in 50 cc of ethanol and 5 cc of water was heated under reflux for 5 hours. After cooling, the solvent was distilled off under reduced pressure and water was added to the residue. The aqueous layer was acidified with 1N HCl and extracted with chloroform. The extract was dried over magnesium sulfate and then distilled to remove the solvent. The crystals thus obtained were filtered and washed with n-hexane to obtain 8.71 g of 5-(2-chlorophenyl)-2-methyl4-thiazolecarboxylic acid. Yield: 95.0%.

The following compounds were obtained in the same manner as in Reference Example 6:

(1) 5-(2-methylphenyl)-2-phenyl-4-thiazolecarboxylic acid,
(2) 2-methyl-5-(2-methylphenyl)-4-thiazolecarboxylic acid,
(3) 5-(2-chlorophenyl)-2-(N-morpholino)-4-thiazolecarboxylic acid.

Reference Example 7

5-(2-Methylphenyl)-2-phenyl-4-oxazolecarboxylic acid

1) Ethyl 3-(2-methylphenyl)-2-hydroxyimino-3-oxopropionate (compound G)

To a stirred solution of 5.00 g of ethyl 3-(2-methylphenyl)-3-oxopropionate in 5 ml of acetic acid was added dropwise 5 ml of an aqueous solution of 1.76 g of sodium nitrite under ice-cooling. The resulting mixture was stirred at room temperature for 1 hour and water was added, followed by extraction with ethyl acetate. The extract was washed with a saturated aqueous sodium chloride solution and dried over magnesium sulfate. The solvent was distilled off to obtain 5.70 g of a crude extract of compound G. Yield: 100%.

2) Ethyl 2-benzoylamino-3-(2-methylphenyl)-3-oxopropionate (compound H)

To a stirred mixture of 10.17 g of compound G, 25 ml of 30% sulfuric acid and 25 g of ice was added 3.40 g of zinc powder, and the resulting mixture was stirred for 1 hour. The reaction mixture was filtered, after which the filtrate was washed with diethyl ether and the aqueous layer was separated. To the aqueous layer were added 12.3 g of sodium acetate and 1.97 ml of benzoyl chloride, and the resulting mixture was stirred at room temperature for 1 hour. The aqueous layer thus obtained was neutralized with sodium hydrogencarbonate and extracted with chloroform. The extract

was dried over magnesium sulfate and distilled to remove the solvent. The crude product thus obtained was purified by a silica gel column chromatography (eluent: ethyl acetate-n-hexane) to obtain 1.86 g of compound H. Yield: 35.2%.

3) Ethyl 5-(2-methylphenyl)-2-phenyl-4-oxazolecarboxylate (compound I)

To a stirred solution of 0.90 g of compound H in 5 mg of benzene was added 0.70 ml of phosphoryl chloride, and the resulting mixture was refluxed for 3 hours. Into an aqueous sodium hydrogencarbonate solution was poured the mixture, followed by extraction with ethyl acetate. The extract was dried over magnesium sulfate and distilled to remove the solvent. The crude product thus obtained was purified by a silica gel column chromatography (eluent: ethyl acetate-n-hexane) to obtain 0.78 g of compound I. Yield: 91.5%.

4) 5-(2-Methylphenyl)-2-phenyl-4-oxazolecarboxylic acid

A mixture of 0.78 g of compound I and 0.16 g of potassium hydroxide in 10 cc of ethanol and 1 cc of water was heated under reflux for 5 hours. After cooling, the solvent was distilled off under reduced pressure and water was added to the residue. The aqueous layer was acidified with 1N HCl and extracted with chloroform. The extract was dried over magnesium sulfate and then distilled to remove the solvent. The crystals thus obtained were filtered and washed with n-hexane to obtain 0.64 g of 5-(2-methylphenyl)-2-phenyl-4-oxazolecarboxylic acid. Yield: 89.6%. The following compound was obtained in the same manner as in Reference Example 7: 5-(2-chlorophenyl)-2-phenyl-4-oxazolecarboxylic acid.

Reference Example 8

4-(2-Methylphenyl)-2-phenyl-5-oxazolecarboxylic acid

1) Ethyl 2-benzoyloxy-3-(2-methylphenyl)-3-oxopropionate (compound J)

A mixture of 13.83 g of ethyl 2-bromo-3-(2-methylphenyl)-3-oxopropionate, 6.99 g of sodium benzoate and 2 drops of sulfuric acid in 50 ml of ethanol was refluxed for 3 hours. The solvent was distilled off and water was added to the residue, followed by extraction with chloroform. The extract was washed with a saturated aqueous sodium chloride solution and dried over magnesium sulfate. The solvent was distilled off and the crude product thus obtained was purified by a silica gel column chromatography (eluent: ethyl acetate-n-hexane) to obtain 13.27 g of compound J. Yield: 83.8%.

2) Ethyl 4-(2-methylphenyl)-2-phenyl-5-oxazolecarboxylate (compound K)

A mixture of 4.00 g of compound J and 4.25 g of ammonium acetate in 15 cc of acetic acid was refluxed for 3 hours. The acetic acid was distilled off and water was added to the residue, followed by extraction with chloroform. The extract was dried over magnesium sulfate and distilled to remove the solvent. The crude product thus obtained was purified by a silica gel column chromatography (eluent: ethyl acetate-n-hexane) to obtain 0.93 g of compound K. Yield: 24.8%.

3) 4-(2-Methylphenyl)-2-phenyl-5-oxazolecarboxylic acid

A mixture of 0.93 g of compound K and 0.20 g of potassium hydroxide in 10 cc of ethanol and 1 cc of water was heated under reflux for 5 hours. After cooling, the solvent was distilled off under reduced pressure and water was added to the residue. The aqueous layer was acidified with 1N HCl and extracted with chloroform. The extract was dried over magnesium sulfate and then distilled to remove the solvent. The crystals thus obtained were filtered and washed with n-hexane to obtain 0.72 g of 4-(2-methylphenyl)-2-phenyl-5-oxazolecarboxylic acid. Yield: 85.3%.

Reference Example 9

4-(2-Chlorophenyl)-1-methyl-2-phenyl-5-imidazolecarboxylic acid and 5-(2-chlorophenyl)-1-methyl-2-phenyl-4-imidazolecarboxylic acid

1) Ethyl 4(5)-(2-chlorophenyl)-2-phenyl-5(4)-imidazolecarboxylate (compound L)

A mixture of 4.18 g of ethyl 3-(2-methylphenyl)-2,3-dioxopropionate monohydrate, 3.3 ml of benzaldehyde and 12.5 g of ammonium acetate in 50 ml of acetic acid was heated at 70°C for 30 minutes. The solvent was distilled off and water and then ethyl acetate were added to the residue. The organic layer was further washed with an aqueous sodium hydrogencarbonate solution and then a saturated aqueous sodium chloride solution and dried over magnesium sulfate.

The solvent was distilled off and the crude product thus obtained was purified by a silica gel column chromatography (eluent: ethyl acetate-n-hexane) to obtain 1.29 g of compound L. Yield: 24.4%.

2) Ethyl 4-(2-chlorophenyl)-1-methyl-2-phenyl-5-imidazolecarboxylate (compound M) and ethyl 5-(2-chlorophenyl)-1-methyl-2-phenyl-4-imidazolecarboxylate (compound N)

A mixture of 654 mg of compound L, 560 mg of potassium carbonate and 0.19 ml of iodomethane in 10 ml of dimethylformamide was stirred at room temperature for 1 hour. Water was added to the mixture, followed by extraction with ethyl acetate. The extract was washed with water and dried over magnesium sulfate. The solvent was distilled off and the crude product thus obtained was purified by a silica gel column chromatography (eluent: ethyl acetate-n-hexane) to obtain 400 mg (yield 58.7%) of compound M and 243 mg (yield 35.7%) of compound N.

3) 4-(2-Chlorophenyl)-1-methyl-2-phenyl-5-imidazolecarboxylic acid

A mixture of 400 mg of compound M and 132 mg of potassium hydroxide in 4 cc of ethanol and 1 cc of water was heated under reflux for 5 hours. After cooling, the solvent was distilled off under reduced pressure and water was added to the residue. The aqueous layer was acidified with 1N HCl and extracted with chloroform. The extract was dried over magnesium sulfate and then distilled to remove the solvent. The crystals thus obtained were filtered and washed with n-hexane to obtain 253 mg of 4-(2-chlorophenyl)-1-methyl-2-phenyl-5-imidazolecarboxylic acid. Yield: 69.2%.

4) 5-(2-Chlorophenyl)-1-methyl-2-phenyl-4-imidazolecarboxylic acid

A mixture of 243 mg of compound N and 80 mg of potassium hydroxide in 3 cc of ethanol and 0.5 cc of water was heated under reflux for 4 hours. After cooling, the solvent was distilled off under reduced pressure and water was added to the residue. The aqueous layer was acidified with 1N HCl and extracted with chloroform. The extract was dried over magnesium sulfate and then distilled to remove the solvent. The crystals thus obtained were filtered and washed with n-hexane to obtain 144 mg of 5-(2-chlorophenyl)-1-methyl-2-phenyl-4-imidazolecarboxylic acid. Yield: 66.1%.

The following compounds were obtained in the same manner as in Reference Example 9:

(1) 1-benzyl-5-(2-chlorophenyl)-2-phenyl-4-imidazolecarboxylic acid,
(2) 1-benzyl-4-(2-chlorophenyl)-2-phenyl-5-imidazolecarboxylic acid,
(3) 1-benzyl-2,4-diphenyl-5-imidazolecarboxylic acid,
(4) 5-(2-chlorophenyl)-2-phenyl-1-(2-trimethylsilylethoxy)methyl-4-imidazolecarboxylic acid,
(5) 4-(2-chlorophenyl)-2-phenyl-1-(2-trimethylsilylethoxy)methyl-5-imidazolecarboxylic acid,
(6) 5-(2-chlorophenyl)-1-methoxymethyl-2-phenyl-4-imidazolecarboxylic acid,
(7) 4-(2-chlorophenyl)-1-methoxymethyl-2-phenyl-5-imidazolecarboxylic acid,
(8) 5-(2-chlorophenyl)-1-(4-methoxybenzyl)-2-phenyl-4-imidazolecarboxylic acid,
(9) 4-(2-chlorophenyl)-1-(4-methoxybenzyl)-2-phenyl-5-imidazolecarboxylic acid.

In the following formulation examples, parts are all by weight.

Formulation Example 1

A powder was prepared by mixing uniformly and pulverizing or granulating finely the following ingredients:

| | |
|---|---|
| Each compound of the invention | 10 parts |
| Magnesium stearate | 10 parts |
| Lactose | 80 parts |

Formulation Example 2

Granules were prepared by kneading together uniformly, grinding, and granulating the following ingredients, followed by sieving:

| | |
|---|---|
| Each compound of the invention | 50 parts |
| Starch | 10 parts |
| Lactose | 15 parts |
| Ethyl cellulose | 20 parts |
| Poly(vinyl alcohol) | 5 parts |
| Water | 30 parts |

Formulation Example 3

Tablets with a diameter of 10 mm were prepared by mixing 99 parts of the granules obtained in Formulation Example 2 with 1 part of calcium stearate, and compression-molding the resulting mixture.

Formulation Example 4

Granules were prepared in the same manner as in Formulation Example 2 except for using the following ingredients:

| | |
|---|---|
| Each compound of the invention | 95 parts |
| Poly(vinyl alcohol) | 5 parts |
| Water | 30 parts |

To 90 parts of the granules obtained was added 10 parts of crystalline cellulose, and the resulting mixture was compression-molded into tablets with a diameter of 8 mm. Then, the tablets were made into dragée by the use of suitable amounts of a mixed suspension of syrup, gelatin and precipitated calcium carbonate and a coloring agent.

Formulation Example 5

An injection was prepared by mixing by heating, and then sterilizing the following ingredients:

| | |
|---|---|
| Each compound of the invention | 0.5 part |
| Nonionic surfactant | 2.5 parts |
| Physiological saline | 97 parts |

Formulation Example 6

Capsules were prepared by packing the powder obtained in Formulation Example 1 into commercially available capsular containers.

Next, test examples are described below for proving the effectiveness of the present invention.

Test Example 1

Inhibitory activity on acyl-CoA:cholesterol acyltransferase (ACAT)

The enzyme used in the test was prepared according to the method of Heider et al. [J. Lipid Res. 24, 1127 (1983)].

A white rabbit was killed by blood-letting under anesthesia, after which its intestinal mucosa was collected and then homogenized, and microsomal fraction was obtained by stepwise centrifugation. The microsomal fraction was suspended in 0.154 M phosphate buffer (pH 7.4) and stored at -80°C until use.

ACAT activity was determined by a modification of the method of Helgerud et al. [J. Lipid Res. 22, 271 (1981)] by measuring radioactivity incorporated into cholesterol esters from [1-$^{14}$C]oleyl-CoA, as an indication. As to the ACAT-inhibitory activity of each compound to be tested, the inhibition rate was calculated by the following equation. The results obtained are shown in Table 2.

$$\text{Inhibition rate (\%)} = \frac{\begin{bmatrix}\text{ACAT activity of}\\ \text{control group which}\\ \text{was given solvent}\end{bmatrix} - \begin{bmatrix}\text{ACAT activity of group}\\ \text{group treated with}\\ \text{compound to be tested}\end{bmatrix}}{\begin{bmatrix}\text{ACAT activity of control}\\ \text{group which was given solvent}\end{bmatrix}} \times 100$$

Table 2

| Compound No. | Inhibition rate % | | Compound No. | Inhibition rate % | |
|---|---|---|---|---|---|
| | 1 | 0.01 µm | | 1 | 0.01 µm |
| 1 | 99.9 | 38.7 | 24 | 97.9 | |
| 2 | 99.8 | 55.2 | 25 | | 88.1 |
| 3 | 92.1 | | 26 | | 51.4 |
| 4 | | 63.9 | 27 | | 86.5 |
| 5 | 97.8 | | 28 | | 64.1 |
| 6 | 46.1 | | 29 | | 90.2 |
| 8 | 99.1 | | 30 | | 76.8 |
| 9 | 49.4 | | 31 | | 70.6 |
| 11 | 99.2 | 42.9 | 33 | | 62.0 |
| 12 | 96.2 | | 35 | 99.6 | 33.5 |
| 13 | 72.7 | | 36 | 79.5 | |
| 15 | 99.2 | | 37 | | 80.4 |
| 17 | 99.8 | | 40 | 99.4 | |
| 19 | 99.9 | | 42 | 99.2 | |
| 21 | 99.8 | | 44 | 99.2 | |
| 23 | 99.6 | 59.4 | 45 | 77.9 | |

Test Example 2

Serum cholesterol lowering activity in rats fed on a high-cholesterol diet

Male Sprague-Dawley rats of 6-week-old were divided into three groups. The first group (normal group) was fed an ordinary diet for 4 days. The second group (control group) was fed a high cholesterol diet (containing 1.0% cholesterol, 1.0% cholic acid and 8.5% coconut oil) for 4 days. The third group (treated group) was fed a high cholesterol diet and was treated with a compound for 4 days.

Simultaneously with the beginning of the above feeding, the compound was suspended in a 0.5% carboxymethyl cellulose solution and administered to the treated group, in a dose of 30 mg (in terms of the compound) per kg of body

weight per day for 4 days. A 0.5% carboxymethyl cellulose solution was also administered to the normal group and the control group in the same manner as above.

After 24 hours of the last administration, blood was collected and the cholesterol concentration in serum was measured by an enzymatic method. The reduction rate of the total serum cholesterol concentration was calculated from values obtained for the three groups by the following equation. The results obtained are shown in Table 3.

$$\text{Choresterol reduction rate (\%)} = \frac{(A) - (B)}{(A) - (C)} \times 100$$

wherein

A: the serum cholesterol concentration of the control group.
B: the serum cholesterol concentration of the treated group.
C: the serum cholesterol concentration of the normal group.

Table 3

| Compound No. | Reduction rate (%) | Compound No. | Reduction rate (%) |
|---|---|---|---|
| 1 | 53.8 | 28 | 87.8 |
| 2 | 85.7 | 29 | 46.1 |
| 11 | 46.2 | 30 | 75.7 |
| 23 | 107.7 | 31 | 59.8 |
| 25 | 39.1 | 33 | 73.2 |
| 27 | 61.7 | 37 | 59.0 |

The compounds of the present invention have ACAT-inhibitory activity and are useful as a prophylactic and therapeutic agent for hypercholesterolemia, atherosclerosis and various diseases caused by them.

**Claims**

1. An azole derivative represented by the general formula (I):

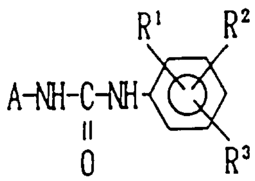

(I).

[wherein $R^1$, $R^2$ and $R^3$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different, and A is a group represented by the formula (i):

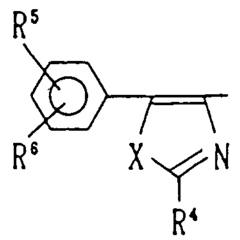

(i)

(wherein $R^4$ is a $C_{1-8}$alkyl group, a $C_{3-8}$cycloalkyl group, a mono-$C_{1-8}$alkylamino group, a di-$C_{1-8}$alkylamino group whose $C_{1-8}$alkyl groups may be the same or different, an unsubstituted aliphatic cyclic amino group, a substituted aliphatic cyclic amino group having one or more $C_{1-8}$alkyl groups as the substituent(s), an unsubstituted phenyl group, or a substituted phenyl group having one or two substituents which may be the same or different and are selected from the group consisting of halogen atoms and $C_{1-8}$alkyl groups; $R^5$ and $R^6$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different; and X is -O-, -S- or -N($R^7$)- (wherein $R^7$ is a hydrogen atom; a $C_{1-8}$alkyl group; an unsubstituted phenyl-$C_{1-8}$alkyl group; a substituted phenyl-$C_{1-8}$alkyl group having one or more substituents which may be the same or different and are selected from the group consisting of halogen atoms, $C_{1-8}$alkyl groups and $C_{1-8}$alkoxy groups; a (2-trimethylsilyl)ethoxymethyl group; or a substituted $C_{1-8}$alkyl group having one or more $C_{1-8}$alkoxy groups as the substituent(s))) or a group represented by the formula (ii):

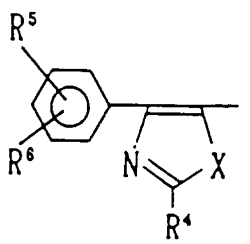

(ii)

(wherein $R^4$, $R^5$, $R^6$ and X are as defined above), provided that when A is a group represented by the formula (ii), there is excluded the case wherein X is -S-, each of $R^1$, $R^2$, $R^3$, $R^5$ and $R^6$ is a hydrogen atom, and $R^4$ is a $C_{1-8}$alkyl group, an unsubstituted phenyl group or a substituted phenyl group having one or two $C_{1-8}$alkyl groups as the substituent(s)] or a pharmacologically acceptable salt thereof.

**2.** An azole derivative or a pharmacologically acceptable salt thereof according to claim 1, wherein $R^1$, $R^2$ and $R^3$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different, and A is a group represented by the formula (i):

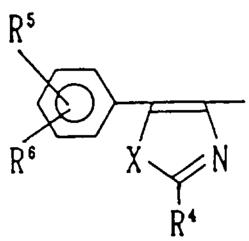

(i)

wherein $R^4$ is a $C_{1-8}$alkyl group, a $C_{3-8}$cycloalkyl group, a di-$C_{1-8}$alkylamino group whose $C_{1-8}$alkyl groups may be the same or different, an N-morpholino group, an N-piperidino group or an unsubstituted phenyl group, $R^5$ is a hal-

ogen atom, a $C_{1-4}$alkyl group or a $C_{1-4}$alkylthio group, $R^6$ is a hydrogen atom, and X is -O-, -S- or -N($R^7$)- (wherein $R^7$ is a hydrogen atom, a $C_{1-4}$alkyl group, an unsubstituted benzyl group, a substituted benzyl group having one or more methoxy groups as the substituent(s), a (2-trimethylsilyl)ethoxymethyl group, or a substituted $C_{1-4}$alkyl group having one or more $C_{1-4}$alkoxy groups as the substituent(s)).

**3.** An azole derivative or a pharmacologically acceptable salt thereof according to any one of claims 1 and 2, wherein $R^1$ and $R^2$, which may be the same or different, are $C_{1-4}$alkyl groups, $R^3$ is a hydrogen atom, and A is a group represented by the formula (i):

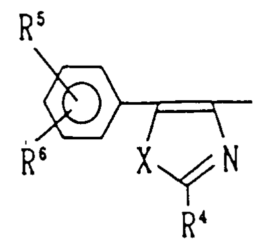

(i)

wherein $R^4$ is a $C_{1-4}$alkyl group or an unsubstituted phenyl group, $R^5$ is a halogen atom, a $C_{1-4}$alkyl group or a $C_{1-4}$alkylthio group, which is a substituent at the ortho position , $R^6$ is a hydrogen atom, and X is -O-, -S- or -N($R^7$)- (wherein $R^7$ is a benzyl group).

**4.** A medicinal composition comprising as an active ingredient an azole derivative or a pharmacologically acceptable salt thereof, according to any one of claims 1, 2 and 3.

**5.** A medicinal composition according to claim 4, which is an acyl-CoA:cholesterol O-acyltransferase inhibitor.

**6.** A process for producing an azole derivative represented by the general formula (I):

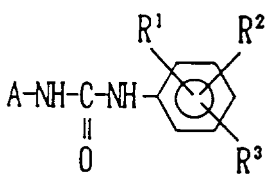

(I)

[wherein $R^1$, $R^2$ and $R^3$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different, and A is a group represented by the formula (i):

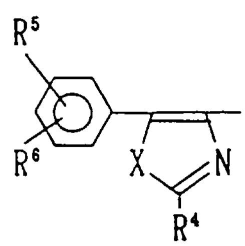

(i)

(wherein $R^4$ is a $C_{1-8}$alkyl group, a $C_{3-8}$cycloalkyl group, a mono-$C_{1-8}$alkylamino group, a di-$C_{1-8}$alkylamino group whose $C_{1-8}$alkyl groups may be the same or different, an unsubstituted aliphatic cyclic amino group, a substituted aliphatic cyclic amino group having one or more $C_{1-8}$alkyl groups as the substituent(s), an unsubstituted phenyl group, or a substituted phenyl group having one or two substituents which may be the same or different and are

selected from the group consisting of halogen atoms and $C_{1-8}$alkyl groups; $R^5$ and $R^6$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different; and X is -O-, -S- or -N($R^7$)- (wherein $R^7$ is a hydrogen atom; a $C_{1-8}$alkyl group; an unsubstituted phenyl-$C_{1-8}$alkyl group; a substituted phenyl-$C_{1-8}$alkyl group having one or more substituents which may be the same or different and are selected from the group consisting of halogen atoms, $C_{1-8}$alkyl groups and $C_{1-8}$alkoxy groups; a (2-trimethylsilyl)ethoxymethyl group; or a substituted $C_{1-8}$alkyl group having one or more $C_{1-8}$alkoxy groups as the substituent(s))) or a group represented by the formula (ii):

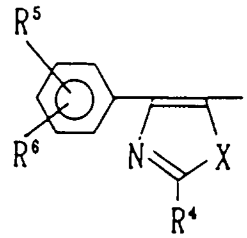

(ii)

(wherein $R^4$, $R^5$, $R^6$ and X are as defined above), provided that when A is a group represented by the formula (ii), there is excluded the case wherein X is -S-, each of $R^1$, $R^2$, $R^3$, $R^5$ and $R^6$ is a hydrogen atom, and $R^4$ is a $C_{1-8}$alkyl group, an unsubstituted phenyl group or a substituted phenyl group having one or two $C_{1-8}$alkyl groups as the substituent(s)] which comprises reacting a compound represented by the general formula (II):

$$A\text{-}COOH \qquad (II)$$

(wherein A is as defined above) with diphenylphosphoryl azide to form a compound represented by the general formula (III):

$$A\text{-}NCO \qquad (III)$$

(wherein A is as defined above), and then reacting the compound (III) with a compound represented by the general formula (IV):

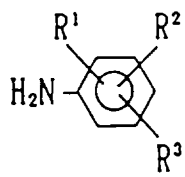

(IV)

(wherein $R^1$, $R^2$ and $R^3$ are as defined above).

7. A process for producing an azole derivative represented by the general formula (I):

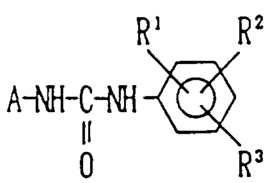

(I)

[wherein $R^1$, $R^2$ and $R^3$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different, and A is a group represented by the formula (i):

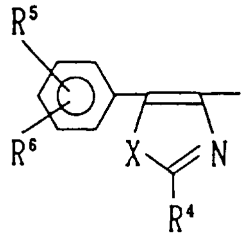

(i)

(wherein $R^4$ is a $C_{1-8}$alkyl group, a $C_{3-8}$cycloalkyl group, a mono-$C_{1-8}$alkylamino group, a di-$C_{1-8}$alkylamino group whose $C_{1-8}$alkyl groups may be the same or different, an unsubstituted aliphatic cyclic amino group, a substituted aliphatic cyclic amino group having one or more $C_{1-8}$alkyl groups as the substituent(s), an unsubstituted phenyl group, or a substituted phenyl group having one or two substituents which may be the same or different and are selected from the group consisting of halogen atoms and $C_{1-8}$alkyl groups; $R^5$ and $R^6$, which may be the same or different, are hydrogen atoms, halogen atoms, $C_{1-8}$alkyl groups, halo-$C_{1-8}$alkyl groups, $C_{1-8}$alkoxy groups, $C_{1-8}$alkylthio groups, mono-$C_{1-8}$alkylamino groups, or di-$C_{1-8}$alkylamino groups whose $C_{1-8}$alkyl groups may be the same or different; and X is -O-, -S- or -N($R^7$)- (wherein $R^7$ is a hydrogen atom; a $C_{1-8}$alkyl group; an unsubstituted phenyl-$C_{1-8}$alkyl group; a substituted phenyl-$C_{1-8}$alkyl group having one or more substituents which may be the same or different and are selected from the group consisting of halogen atoms, $C_{1-8}$alkyl groups and $C_{1-8}$alkoxy groups; a (2-trimethylsilyl)ethoxymethyl group; or a substituted $C_{1-8}$alkyl group having one or more $C_{1-8}$alkoxy groups as the substituent(s))) or a group represented by the formula (ii):

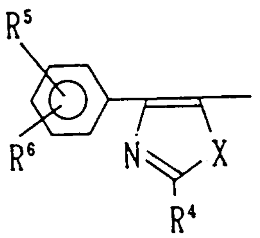

(ii)

(wherein $R^4$, $R^5$, $R^6$ and X are as defined above), provided that when A is a group represented by the formula (ii), there is excluded the case wherein X is -S-, each of $R^1$, $R^2$, $R^3$, $R^5$ and $R^6$ is a hydrogen atom, and $R^4$ is a $C_{1-8}$alkyl group, an unsubstituted phenyl group or a substituted phenyl group having one or two $C_{1-8}$alkyl groups as the substituent(s)] which comprises reacting a compound represented by the general formula (V):

$$A\text{-}NH_2 \qquad (V)$$

(wherein A is as defined above) with a compound of the general formula (VI):

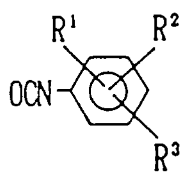

(VI)

(wherein $R^1$, $R^2$ and $R^3$ are as defined above).

**8.** A method for using a medicinal composition which comprises using as an acyl-CoA:cholesterol O-acyltransferase inhibitor a medicinal composition comprising as an active ingredient an azole derivative or a pharmacologically acceptable salt thereof, according to claim 4.

European Patent Office

# EUROPEAN SEARCH REPORT

Application Number
EP 96 11 4020

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| X,D | US-A-5 162 360 (CRESWELL MARK W ET AL) 10 November 1992<br>* see col.2, formula 1 and 2, col.3, formula 3,6,7 and 9 *<br>* the whole document *<br>--- | 1-7 | C07D277/46<br>C07D263/34<br>C07D233/88<br>A61K31/17<br>A61K31/42<br>A61K31/425<br>A61K31/40 |
| X | CURR.MED.CHEM.,<br>vol. 1, no. 3, 1994,<br>pages 204-225, XP002021008<br>SLISKOVIC,D.R. ET AL.: "ACAT Inhibitors : Potential Anti-atherosclerotic Agents "<br>* see pages 216/217, Tables 10 - 13 *<br>--- | 1-7 | |
| Y | US-A-5 185 358 (CRESWELL MARK W ET AL) 9 February 1993<br>* the whole document *<br>--- | 1-7 | |
| Y | EP-A-0 405 233 (MITSUBISHI CHEM IND) 2 January 1991<br>* the whole document *<br>--- | 1-7 | TECHNICAL FIELDS SEARCHED (Int.Cl.6) |
| Y | EP-A-0 506 532 (LIPHA) 30 September 1992<br>* the whole document *<br>--- | 1-7 | C07D |
| Y | EP-A-0 613 894 (NIHON NOHYAKU CO LTD) 7 September 1994<br>* the whole document *<br>--- | 1-7 | |
| Y | J.MED.CHEM.,<br>vol. 36, no. 22, 1993, WASHINGTON,<br>pages 3300-3307, XP002021009<br>TRIVEDI,B.K. ET AL.: "Inhibitors of Acyl-CoA:Cholesterol Acyltransferase. 4. A Novel Series of Urea ACAT Inhibitors as Potential Hypocholesterolemic Agents "<br>* see page 3303,Table V, and fig. 2 *<br>---<br>-/-- | 1-7 | |

The present search report has been drawn up for all claims

| Place of search | Date of completion of the search | Examiner |
|---|---|---|
| MUNICH | 12 December 1996 | Stellmach, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 96 11 4020

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 113 731 (WINTERS GIORGIO ET AL) 12 September 1978<br>* the whole document *<br>----- | 1-7 | |
| | | | TECHNICAL FIELDS SEARCHED (Int.Cl.6) |

The present search report has been drawn up for all claims

| Place of search | Date of completion of the search | Examiner |
|---|---|---|
| MUNICH | 12 December 1996 | Stellmach, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)